(19) Europäisches Patentamt
European Patent Office
Office européen des brevets

(11) Veröffentlichungsnummer: **0 000 487**
**A1**

(12) # EUROPÄISCHE PATENTANMELDUNG

(21) Anmeldenummer: **78100326.4**

(22) Anmeldetag: **07.07.78**

(51) Int. Cl.²: **C 07 D 211/00**, C 07 D 491/00, C 08 K 5/00, C 08 L 23/06, C 07 D 401/00

(30) Priorität: **15.07.77 CH 8793/77**

(43) Veröffentlichungstag der Anmeldung: **07.02.79 Bulletin 79/3**

(84) Benannte Vertragsstaaten: **BE CH DE FR GB NL**

(71) Anmelder: **CIBA-GEIGY AG**
**Patentabteilung Postfach**
**CH-4002 Basel. (CH)**

(72) Erfinder: **Moser, Paul, Dr.**
**Leimgrubenweg 56**
**CH-4125 Riehen. (CH)**

(54) **Enolgruppen enthaltende Verbindungen, deren Metallchelate, deren Herstellung und Verwendung als Lichtschutzmittel insbesondre in hockmolekularen Verbindungen.**

(57) Die Verbindungen der allgemeinen Formel I

$$M^{q\oplus}\left[L^{r\ominus}\right]_{q/r} \cdot mA \qquad (I),$$

worin

$M^{q\oplus}$ ein zweifach oder dreifach positiv geladenes Metallion ist, und
q 2 oder 3 ist, und
r 1 oder 2 ist, wobei der Quotient q/r 1,1.5,2 oder 3 ist, und
L ein durch mindestens eine Enolatgruppe in 1-oder 4-Steilung substituiertes Piperidin dessen Ringsticksoff durch Alkylsubstituenten an den benachbarten Stellungen sterisch gehindert ist bedeuten, und
m 0, 1 oder 2 ist, und
A $H_2O$ oder ein Amin ist.

sind wertvolle Lichtschutzmittel insbesondere für höhermolekulare Verbindungen.

EP 0 000 487 A1

3-11250/S/+

BEZEICHNUNG GEÄNDERT
siehe Titelseite

Neue Lichtschutzmittel

Die vorliegende Erfindung betrifft neue, Enolgruppen enthaltende, Verbindungen, deren Metallchelate, deren Herstellung und Verwendung als Lichtschutzmittel in organischem Material, sowie das mit deren Hilfe geschützte organische Material.

In der DT-OS 2,625,967 sind Metallkomplexe mit sterisch gehinderten Aminen und einfach geladenen Anionen als Stabilisatoren für synthetische Polymere beschrieben worden.

Es wurden nun neue, Enolgruppen enthaltende, Verbindungen, sowie deren Chelatkomplexe mit zwei- und dreifach geladenen Metallkationen gefunden, welche sich durch gute Lichtschutzwirkung und gute Extraktionsstabilität auszeichnen und gute Verträglichkeit in Polymeren besitzen.

Die neuen Verbindungen entsprechen der allgemeinen Formel I

$$M^{q\oplus} \left[L^{r\ominus}\right]_{q/r} \cdot m\,A \qquad (I),$$

worin

$M^{q\oplus}$ ein zweifach oder dreifach positiv geladenes Metallion ist, und

q 2 oder 3 ist, und

r 1 oder 2 ist, wobei der Quotient q/r 1, 1.5, 2 oder 3 ist, und

L ein durch mindestens eine Enolatgruppe in 1- oder 4-Stellung substituierter Piperidin, dessen Ring-Stickstoff durch Alkylsubstituenten an den benachbarten Stellungen sterisch gehindert ist, bedeutet, und

m 0, 1 oder 2 ist, und

A $H_2O$ oder ein Amin ist.

Die vorliegende Erfindung betrifft vor allem Verbindungen der Formel I, worin

$M^{q\oplus}$ ein zweifach oder dreifach positiv geladenes Metallion ist, und

q 2 oder 3 ist, und

r 1, oder 2 ist, wobei der Quotient q/r 1, 1.5, 2 oder 3 bedeutet, und

L eine Gruppe der Formel II

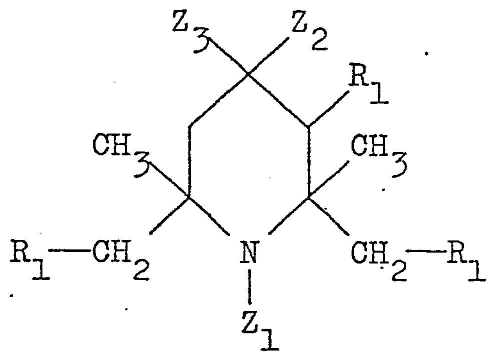

(II)

ist, worin

$R_1$ Wasserstoff oder $C_1$-$C_4$ Alkyl ist, und

$Z_1$ Wasserstoff, $C_1$-$C_{12}$ Alkyl, $C_3$-$C_8$ Alkenyl, $C_3$-$C_4$ Alkinyl, $C_2$-$C_{21}$ Alkoxyalkyl, $C_7$-$C_8$ Aralkyl, eine aliphatische Acylgruppe mit 1-4 C-Atomen, oder eine der Gruppen $-CH_2COOR_2$ oder $-CH_2-CH(R_3)-OR_4$, oder einen Rest $-(CH_2)_4-R_5$, $-CH_2-CH=CH-CH_2-R_5$, $-CH_2-$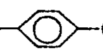$-CH_2-R_5$, $-CH_2-$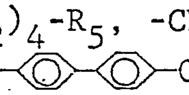$-CH_2-R_5$, oder eine Gruppe der Formel III

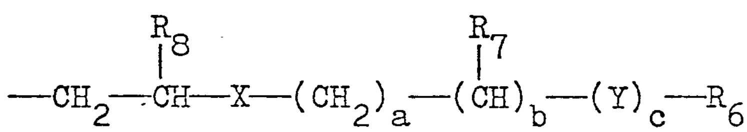

(III)

bedeutet, wobei

$R_2$ $C_1$-$C_8$ Alkyl, $C_3$-$C_6$ Alkenyl, Phenyl, $C_7$-$C_8$ Aralkyl oder Cyclohexyl ist, und

$R_3$ Wasserstoff, Methyl oder Phenyl ist, und

$R_4$ eine aliphatische $C_1$-$C_{18}$ Acylgruppe, eine aromatische $C_7$ Acylgruppe, eine araliphatische $C_8$-$C_9$ Acylgruppe oder eine alicyclische $C_6$-$C_9$ Acylgruppe bedeutet, wobei der aromatische Teil gegebenenfalls mit Chlor, $C_1$-$C_4$ Alkyl, $C_1$-$C_8$ Alkoxy, und/oder Hydroxy substituiert sein kann bedeutet, und

$R_5$ eine Gruppe der Formel IV

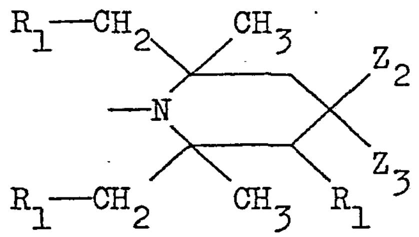

(IV)

bedeutet, worin $R_1$, $Z_2$ und $Z_3$ die hier angegebene Bedeutung haben, und

$R_6$ eine Gruppe der Formel V

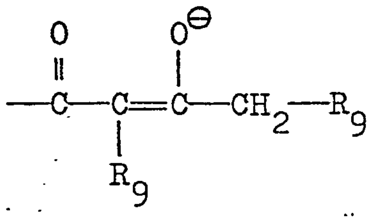

(V)

ist, worin

$R_9$ Wasserstoff oder $C_1$-$C_4$ Alkyl ist, und

$R_7$ und $R_8$ unabhängig voneinander Wasserstoff, Methyl, Phenyl, $C_2$-$C_9$ Alkoxymethyl oder ein Rest $-CH_2O-R_6$ ist, worin $R_6$ die oben definierte Bedeutung hat, und

a 0, 1 oder 2, und

b 0 oder 1 ist, und

c 0 oder, falls a und/oder b von 0 verschieden sind, auch 1 ist, und

X und Y unabhängig voneinander -O- oder $-NR_{10}$, worin

$R_{10}$ Wasserstoff, $C_1$-$C_{12}$ Alkyl, $C_3$-$C_8$ Alkenyl, Cyclohexyl, $C_3$-$C_{21}$ Alkoxyalkyl, $C_7$-$C_{13}$ Aralkyl, gegebenenfalls durch $C_1$-$C_8$ Alkyl und/oder $C_1$-$C_8$ Alkoxy substituiertes Phenyl oder eine Gruppe $-CH_2-CH_2-O-R_6$, worin $R_6$ die oben angegebene Bedeutung hat, ist, und $R_{10}$ ferner eine Gruppe der Formel VI

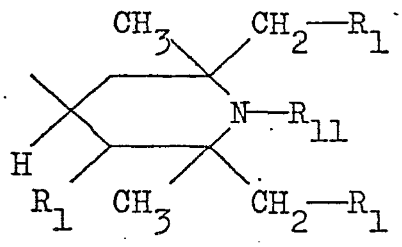

(VI) ,

worin

$R_1$ die oben angegebene Bedeutung hat, und

$R_{11}$ Wasserstoff, $C_1$-$C_{12}$ Alkyl, $C_3$-$C_8$ Alkenyl, $C_2$-$C_{21}$ Alkoxyalkyl, $C_7$-$C_8$ Aralkyl, eine aliphatische Acylgruppe mit 1-4 C-Atomen oder eine Gruppe $-CH_2COOR_2$, worin $R_2$ die oben definierte Bedeutung hat, bedeutet, und

$Z_2$ Wasserstoff, $C_2$-$C_{18}$ aliphatisches Acyloxy, gegebenenfalls durch $C_1$-$C_8$ Alkyl oder $C_1$-$C_8$ Alkoxy substituiertes Benzoyloxy oder eine gegebenenfalls $C_1$-$C_8$ N-alkyl-substituierte $C_2$-$C_{18}$ Acylamidogruppe ist, oder eine der Gruppen $-R_{12}$, $-(CH_2)_d-R_{12}$, 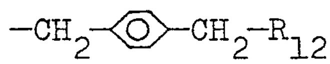 oder 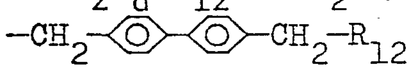 ist, worin d 2 bis 10 ist und $R_{12}$ eine Gruppe der Formel VII

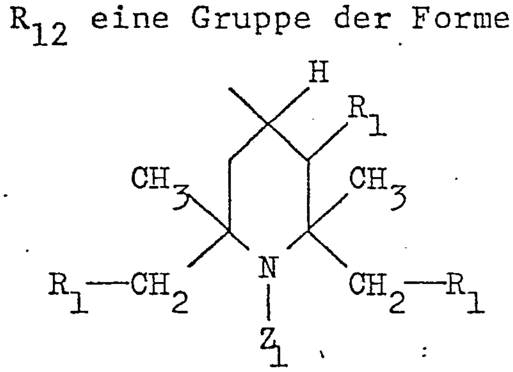

(VII)

ist, worin

$R_1$ und $Z_1$ die oben angegebene Bedeutung haben, oder

$Z_2$ ferner eine Gruppe der Formel VIII

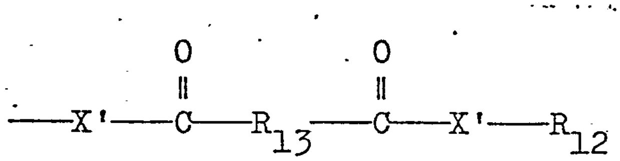 (VIII) ,

darstellt, worin

$R_{12}$ die oben angegebene Bedeutung hat und

X' -O- oder 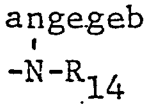 ist, wobei

$R_{14}$ Wasserstoff oder $C_1$-$C_4$ Alkyl bedeutet, und

$R_{13}$ $-(CH_2)_e-$ oder eine Gruppe

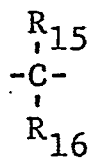 ist, wobei e 0 bis 8 ist, und

$R_{15}$ und $R_{16}$ unabhängig voneinander Wasserstoff, $C_1$-$C_{12}$ Alkyl, $C_3$-$C_8$ Alkenyl, $C_7$-$C_{11}$ Aralkyl, Cyanomethyl, Cyanoäthyl oder eine Gruppe $-CH_2COOR_{17}$, wobei

$R_{17}$ Methyl oder Aethyl ist, bedeuten, oder

$Z_2$ eine der Gruppen

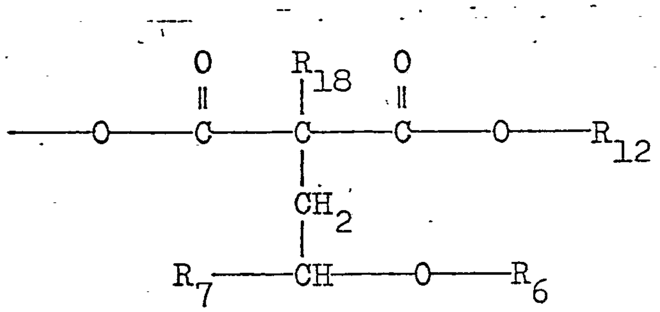 (IX)

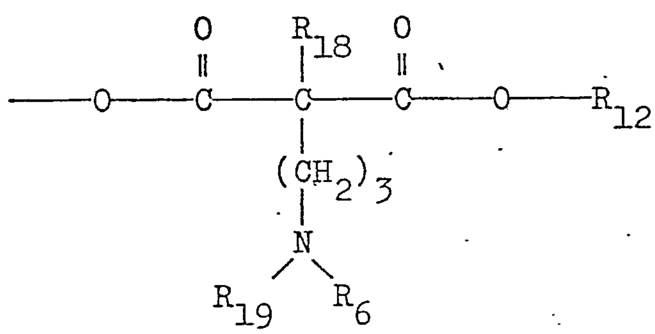 (X) oder

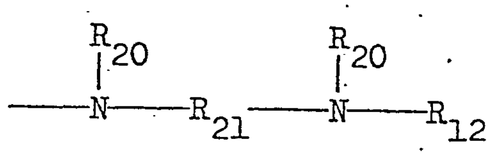 (XI) bedeutet

worin

$R_6$, $R_7$ und $R_{12}$ die oben angegebene Bedeutung haben, und

$R_{18}$ Wasserstoff, $C_1$-$C_8$ Alkyl, Allyl, Phenyl oder Benzyl ist, und

$R_{19}$ Wasserstoff, $C_1$-$C_8$ Alkyl, Allyl oder Benzyl ist, und

$R_{20}$ $C_1$-$C_4$ Alkyl oder eine Gruppe der Formel V mit oben angegebener Bedeutung ist und

$R_{21}$ $C_2$-$C_6$ Alkylen, Cyclohexyliden, Phenylen, Diphenylen, 4,4'-Diphenylenoxyd oder 4,4'-Diphenylenmethan ist, oder

$Z_2$ ferner eine Gruppe der Formeln XII, XIII oder XIV

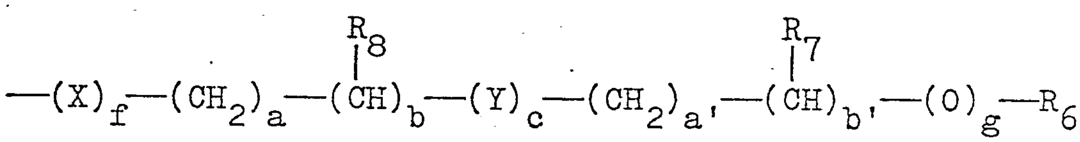

(XII)

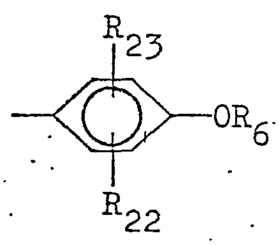 (XIII) oder 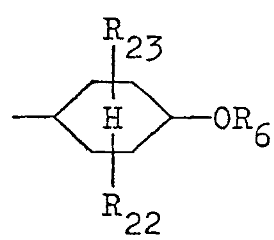 (XIV)

ist, worin a, b, c, X, Y, $R_6$, $R_7$ und $R_8$ die oben angegebene Bedeutung haben, und

f, a' und b' 0 oder 1 sind, und

g 0 oder falls a' und/oder b' von 0 verschieden sind, auch 1 ist, und

$R_{22}$ und $R_{23}$ unabhängig voneinander Wasserstoff, $C_1$-$C_4$ Alkyl, Cyclohexyl, Phenyl, Benzyl, α-Methylbenzyl oder

α,α-Dimethylbenzyl bedeuten, und

$Z_3$ Wasserstoff oder Cyano ist, oder

$Z_2$ und $Z_3$ zusammen eine der Gruppen XV, XVI oder XVII

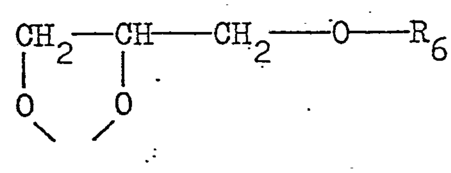

(XV)

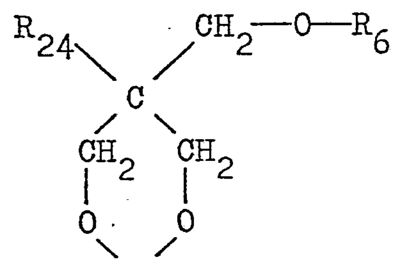

(XVI) oder

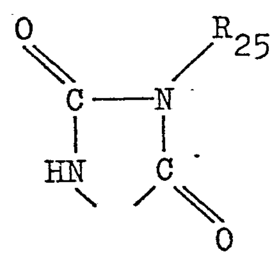

(XVII) bedeuten, worin

$R_6$ oben angegebene Bedeutung hat, und

$R_{24}$ Wasserstoff, Methyl oder Aethyl ist, und

$R_{25}$ Wasserstoff, $C_1$-$C_{18}$ Alkyl, $C_3$-$C_8$ Alkenyl, Cyclohexyl, $C_7$-$C_8$ Aralkyl oder eine Gruppe $-CH_2-COOR_{26}$ oder $-CH_2-CH(R_8)-O-R_6$, bedeutet, wobei

$R_{26}$ $C_1$-$C_{18}$ Alkyl ist, und $R_6$ und $R_8$ die oben angegebene Bedeutung haben, und

m 0, 1 oder 2 ist, und

A $H_2O$ oder ein Amin der Formel (XVIII)

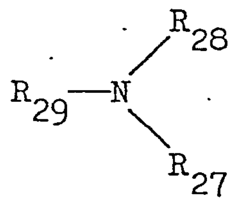

(XVIII)

ist, worin

$R_{27}$ gegebenenfalls substituiertes Alkyl, Cycloalkyl, Aryl, Aralkyl, eine gegebenenfalls substituierte Aminoalkylgruppe oder eine Piperidinylgruppe ist, und

$R_{28}$ Wasserstoff oder gegebenenfalls substituiertes Alkyl, Cycloalkyl, eine gegebenenfalls substituierte Aminoalkylgruppe oder eine gegebenenfalls substituierte Piperidinylgruppe bedeutet, oder

$R_{27}$ und $R_{28}$ zusammen mit dem N-Atom einen durch Alkylgruppen substituierten oder unsubstituierten Pyrrolidin-, Piperidin- oder Morpholinring bilden, und

$R_{29}$ Wasserstoff oder gegebenenfalls substituiertes Alkyl bedeutet.

Ein Kation M der Wertigkeit q, ist z.B. ein solches aus der Reihe $Mg^{2+}$, $Ca^{2+}$, $Sr^{2+}$, $Ba^{2+}$, $Zn^{2+}$, $Cd^{2+}$, $Al^{3+}$, $Sn^{2+}$, $Cr^{3+}$, $Co^{2+}$, $Ni^{2+}$, ein Oxokomplex von Metallionen, insbesondere $VO^{2+}$ und $MoO_2{}^{2+}$ oder ein Zinnalkylion der Formel $(R^*)_2Sn^{2+}$, oder $(CH_2CH_2COOR^*)_2Sn^{2+}$, worin R* ein $C_1$-$C_8$ Alkyl bedeutet, insbesondere jedoch Aethyl, n-Propyl, und vor allem n-Butyl; bevorzugt als Kationen $M^{q+}$ sind insbesondere $Ca^{2+}$, $Mg^{2+}$, $Zn^{2+}$, $Co^{2+}$, und vor allem $Ni^{2+}$ oder $Al^{3+}$. Die Koordinationszahlen dieser Kationen sind dem Fachmann bekannt, und betragen je nach Metall 4 oder 6.

q ist 2 oder 3, insbesondere 2.

r ist 1 oder 2 und entspricht der Anzahl Enolatanionen im Liganden L. Enthält der Ligand L eine Enolatgruppe so enthält der Komplex in der Regel q Liganden L, falls nicht bei der Herstellung ein Unterschuss an L verwendet worden ist. Enthält der Ligand L zwei Enolatgruppen, so müssen nicht beide enolischen Koordinationsstellen mit ein und demselben Metallzentrum $M^{q+}$ besetzt sein. Zwei Enolatgruppen enthaltende Liganden L eignen sich daher zur Herstellung von oligomeren oder gar polymeren Molekülen, welche sich durch erhöhte Extraktionsstabilität auszeichnen. Das in der Formel I gegebene Verhältnis von Metall $M^{q+}$ zu Ligand $L^{r-}$ stimmt auch in diesem Falle, wenn es auf den dem Polymeren zugrunde liegenden Baustein berechnet wird. Solche Verbindungen sind neu und besitzen ein Molekulargewich von ca. 400-10'000, bevorzugt von 400-2'000.

$R_1$ ist als $C_1$-$C_4$ Alkyl verzweigtes oder insbesondere unverzweigtes Alkyl, wie Aethyl, n-Propyl oder n-Butyl, vor allem aber Methyl. Bevorzugt ist $R_1$ Wasserstoff. Bevorzugt haben alle $R_1$ die gleiche Bedeutung.

$Z_1$ ist als $C_1$-$C_{12}$ Alkyl z.B. Methyl, Aethyl, n-Propyl, n-Butyl, n-Pentyl, n-Octyl, n-Decyl oder n-Dodecyl. Bevorzugt sind Alkylgruppen mit 1-8, insbesondere solche mit 1-4 C-Atomen und vor allem Methyl.

$Z_1$ ist als $C_3$-$C_8$ Alkenyl beispielsweise Allyl, 2-Butenyl, 2-Hexenyl oder 2-Octenyl, insbesondere Allyl.

$Z_1$ ist als $C_3$-$C_4$ Alkinyl z.B. Propargyl.

Bedeutet $Z_1$ $C_2$-$C_{21}$ Alkoxyalkyl, so kann der Alkylteil 1-3 C-Atome enthalten und der Alkoxy-Teil aus

1-18 C-Atomen bestehen, wie z.B. in Methoxymethyl, Aethoxymethyl, 2-Methoxyäthyl, 2-Aethoxyäthyl, 2-n-Butoxyäthyl, 3-n-Butoxyäthyl, 2-Octoxyäthyl oder 2-Octadecyloxyäthyl. Insbesondere zu erwähnen sind Verbindungen, in denen $Z_1$ eine Alkoxyalkylgruppe mit 2-6 C-Atomen bedeutet.

$Z_1$ ist als $C_7$-$C_8$ Aralkyl z.B. Benzyl oder α-Phenyläthyl.

$Z_1$ ist als aliphatische Acylgruppe mit 1-4 C-Atomen, beispielsweise Formyl, Acetyl, Acryloyl oder Crotonyl, insbesondere Acetyl.

Ist $Z_1$ die Gruppe $-CH_2COOR_2$, so bedeutet $R_2$ als $C_1$-$C_8$ Alkyl, z.B. Methyl, Aethyl, Isopropyl, n-Butyl, Isobutyl, t-Butyl, Isopentyl oder n-Octyl. Bevorzugt ist $R_2$ $C_1$-$C_4$ Alkyl. $R_2$ ist als $C_3$-$C_6$ Alkenyl z.B. Allyl, 2-Butenyl oder 2-Hexenyl. $R_2$ ist als $C_7$-$C_8$ Aralkyl z.B. Benzyl oder α-Phenyläthyl.

Ist $Z_1$ die Gruppe $-CH_2-CH(R_3)-OR_4$, so bedeutet $R_3$ Wasserstoff, Methyl oder Phenyl, insbesondere Wasserstoff. $R_4$ ist als aliphatischer $C_1$-$C_{18}$ Acylrest beispielsweise Formyl, Acetyl, Propionyl, Butyryl, Octanoyl, Dodecanoyl, Stearoyl oder Acryloyl. $R_4$ ist als aromatischer $C_7$ Acylrest Benzoyl und als araliphatischer $C_8$-$C_9$ Acylrest, Cinnamoyl, Phenylacetyl oder Phenylpropionyl. Der aromatische Teil ist gegebenenfalls mit Chlor, $C_1$-$C_4$ Alkyl, wie Methyl, Aethyl, n-Propyl oder t-Butyl oder mit $C_1$-$C_8$ Alkoxy, wie Methoxy, Aethoxy, Butoxy oder Octoxy und/oder Hydroxy substituiert. Substituierte aromatische Acylgruppen sind z.B. Chlorobenzoyl, Toluoyl, Isopropylbenzoyl, 2,4-Dichlorobenzoyl, 4-Methoxybenzoyl, 3-Butoxybenzoyl, 2-Hydroxybenzoyl oder 3,5-Di-t.-butyl-4-hydroxy-benzoyl. Eine araliphatische substituierte Acylgruppe ist beispielsweise β-(3,5-Di-t.-butyl-4-hydroxyphenyl)-propionyl. Ist $R_4$ eine alicyclische $C_6$-$C_9$ Acylgruppe, so kann es sich um Cyclohexylcarbonyl oder 2,4-Dimethylcyclohexylcarbonyl handeln.

Bedeutet $Z_1$ eine der Gruppen $-(CH_2)_4-R_5$, $-CH_2-CH=CH-CH_2-R_5$, 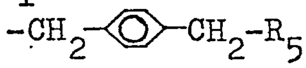 oder 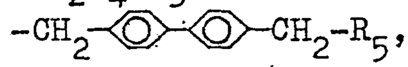, so besitzen die darin enthaltenen Symbole $R_1$, $Z_2$ und $Z_3$ die angegebene Bedeutung. $R_1$ ist in Formel IV besonders bevorzugt Wasserstoff. Bevorzugt werden Verbindungen der Formel I in denen alle im Molekül vorkommenden Reste $R_1$ identisch sind. Dasselbe gilt für alle Reste $Z_2$ und alle Reste $Z_3$.

Bedeutet $Z_1$ eine Gruppe der Formel III, so ist darin $R_6$ eine Gruppe der Formel V, worin $R_9$ $C_1$-$C_4$ Alkyl, wie Aethyl, Propyl, n-Butyl, insbesondere Methyl, und ganz besonders Wasserstoff ist.

$R_7$ und $R_8$ sind als $C_2$-$C_9$ Alkoxymethyl z.B. Methoxymethyl, Aethoxymethyl, Propoxymethyl, Butoxymethyl oder Octoxymethyl. Bevorzugte Bedeutung von $R_7$ und $R_8$ ist Wasserstoff oder Methyl. Besonderes Interesse gilt Verbindungen, in denen $R_7$ Wasserstoff und $R_8$ Wasserstoff oder Methyl bedeutet.

X ist -O- oder $-\overset{|}{N}R_{10}$, bevorzugt -O-.

Y ist -O- oder $-\overset{|}{N}R_{10}$, bevorzugt $-\overset{|}{N}-R_{10}$, wobei $R_{10}$ ganz besonders Wasserstoff oder auch Methyl ist.

$R_{10}$ ist als $C_1$-$C_{12}$ Alkyl z.B. Methyl, Aethyl, n-Propyl, n-Butyl, n-Pentyl, n-Octyl, n-Decyl oder n-Dodecyl, bevorzugt jedoch Methyl.

$R_{10}$ ist als $C_3$-$C_8$ Alkenyl beispielsweise Allyl, 2-Butenyl, 2-Hexenyl oder 2-Octenyl, insbesondere Allyl.

Bedeutet $R_{10}$ $C_3$-$C_{21}$ Alkoxyalkyl, so kann der Alkylteil insbesondere 2-3 C-Atome enthalten und der Alkoxy Teil aus 1-18 C-Atomen bestehen, wie z.B. 2-Methoxyäthyl, 2-Aethoxyäthyl, 2-n-Butoxyäthyl, 3-n-Butoxyäthyl, 2-Octoxyäthyl oder 2-Octadecyloxyäthyl.

$R_{10}$ ist als $C_7$-$C_{13}$ Aralkyl insbesondere Phenyläthyl oder vor allem Benzyl.

Ist $R_{10}$ Phenyl, so kann dieses mit $C_1$-$C_8$ Alkyl wie Methyl, Aethyl, Propyl, Butyl, Hexyl oder Octyl insbesondere Methyl mono- oder disubstituiert sein. $R_{10}$ als Phenyl kann auch mit $C_1$-$C_8$ Alkoxy, wie Methoxy, Aethoxy, Butoxy oder Octoxy, insbesondere Methoxy di- und bevorzugt mono-substituiert sein.

Ist $R_{10}$ eine Gruppe der Formel VI, so kann darin $R_{11}$ als $C_1$-$C_{12}$ Alkyl, $C_3$-$C_8$ Alkenyl, $C_2$-$C_{21}$ Alkoxyalkyl, $C_7$-$C_8$ Aralkyl, oder als aliphatische Acylgruppe mit 1-4 C-Atomen oder als Gruppe $-CH_2COOR$ die oben für $Z_1$ beispielhaft angegebene Bedeutung haben.

$Z_2$ kann bevorzugt Wasserstoff bedeuten.

Ist $Z_2$ $C_2$-$C_{18}$, bevorzugt $C_2$-$C_{12}$ und insbesondere $C_2$-$C_7$ aliphatisches Acyloxy, so kann es sich um Acetyloxy, Propionyloxy, Butyryloxy, Hexanoyloxy, Heptanoyloxy, Dodecanoyloxy oder Stearoyloxy handeln.

$Z_2$ als Benzoyloxy kann mit $C_1$-$C_8$ Alkyl wie Methyl Aethyl, Propyl, Butyl, Octyl, insbesondere mit Methyl, oder mit $C_1$-$C_8$ Alkoxy wie Methoxy, Aethoxy, Butoxy oder Octoxy, insbesondere mit Methoxy, di- und bevorzugt mono-substituiert sein; ganz besonderes Interesse gilt Verbindungen, worin $Z_2$ als Benzoyloxy unsubstituiert ist.

$Z_2$ ist als $C_2$-$C_{18}$ Acylamido, beispielsweise Acetylamido, Propionylamido, Butyrylamido, Hexanoylamido, Heptanoylamido, Dodecanoylamido oder Stearoylamido. Bevorzugt sind Acylamidogruppen mit 2-12, und insbesondere solche mit 2-7 C-Atomen.

Ist $Z_2$ eine $C_1$-$C_8$ N-alkylsubstituierte $C_2$-$C_{18}$ Acylamidogruppe so kommen als N-Alkylsubstituenten z.B. Methyl, Aethyl, n-Propyl, n-Butyl, n-Hexyl oder n-Octyl in Frage.

Enthält $Z_2$ einen Rest $R_{12}$, so kann dieser direkt oder vorzugsweise über eine Alkylengruppe mit 2-10 C-Atomen, bevorzugt 2-6 C-Atomen, wie Dimethylen, Trimethylen, Tetramethylen, Hexamethylen oder Decamethylen mit dem Piperidinring verknüpft sein. Ebenso bevorzugt wie Alkylenbrücken sind auch Verknüpfungen über eine Xylylen oder Bitolylen-Brücke.

$Z_2$ kann eine Gruppe der Formel VIII sein, worin $R_{13}$ als Alkylen beispielsweise 1-8, bevorzugt 1-6 C-Atome besitzen kann, wie Methylen, Dimethylen, Trimethylen, Tetramethylen, Hexamethylen oder Octamethylen. Der Index e kann jedoch ebenso bevorzugt 0 bedeuten, d.h. die Alkylengruppe kann auch fehlen.

X' bedeutet -O- oder $-\overset{|}{N}-R_{14}$, worin $R_{14}$ als $C_1$-$C_4$ Alkyl, Aethyl, Propyl oder Butyl bedeutet, und die bevorzugte Bedeutung von $R_{14}$ Wasserstoff oder Methyl ist.

$R_{13}$ kann auch eine Gruppe $-(R_{15})C(R_{16})-$ sein, wobei $R_{15}$ und $R_{16}$ $C_1$-$C_{12}$, bevorzugt $C_1$-$C_4$ Alkyl, wie Methyl, Aethyl, Propyl, Butyl, Hexyl, Octyl oder Dodecyl sein können.

$R_{15}$ und $R_{16}$ sind als $C_3$-$C_8$ Alkenyl beispielsweise Allyl, 2-Butenyl, 2-Hexenyl oder 2-Octenyl, insbesondere Allyl.

$R_{15}$ und $R_{16}$ sind als $C_7$-$C_{11}$ Aralkyl besonders Phenyläthyl oder insbesondere Benzyl.

$R_{15}$ und $R_{16}$ können Cyanoäthyl und insbesondere auch Cyanomethyl sein.

$R_{18}$ kann in den Gruppen der Formeln IX oder X als $C_1$-$C_8$ Alkyl z.B. Methyl, Aethyl, Propyl, Butyl, Hexyl oder Octyl sein. Bevorzugt ist $R_{18}$ jedoch Wasserstoff.

$R_{19}$ in Formel X kann als $C_1$-$C_8$ Alkyl, bevorzugt $C_1$-$C_4$ Alkyl, beispielsweise Methyl, Aethyl, n-Propyl, n-Butyl, Hexyl oder Octyl sein. Ebenso bevorzugt bedeutet $R_{19}$ auch Wasserstoff, Allyl oder Benzyl.

In Formel XI bedeutet $R_{20}$ als $C_1$-$C_4$ Alkyl z.B. Methyl, Aethyl, n-Propyl oder n-Butyl. Als bevorzugte Bedeutung von $R_{20}$ ist jedoch eine Gruppe der Formel V zu nennen.

$R_{21}$ ist in der Formel XI besonders bevorzugt $C_2$-$C_6$ Alkylen wie Dimethylen, Trimethylen, Tetramethylen oder Hexamethylen. Es kann bevorzugt jedoch auch Cyclohexyliden oder Phenylen bedeuten.

Bedeutet $Z_2$ eine Gruppe der Formel XII, so sind die darin enthaltenen Indices a', b' und g bevorzugt 0.

$R_{22}$ und $R_{23}$ in den Formeln XIII und XIV können als $C_1$-$C_4$ Alkyl z.B. Methyl, Aethyl, Propyl oder Butyl sein, bevorzugt sind Aethyl und besonders Methyl, sowie auch Wasserstoff.

Bilden $Z_2$ und $Z_3$ zusammen einen Heterocyclus so sind vor allem Gruppen der Formel XV und XVI bevorzugt.

$R_{24}$ ist dabei Aethyl, bevorzugt Methyl und insbesondere Wasserstoff.

$R_{25}$ ist als $C_1$-$C_{12}$ Alkyl beispielsweise Methyl, Aethyl, Propyl, Butyl, Hexyl, Octyl, Decyl, oder Dodecyl. Bevorzugt sind Alkylgruppen mit 1-8 C-Atomen.

$R_{25}$ ist als Aralkyl mit 7-8 C-Atomen vor allem Phenyläthyl oder insbesondere Benzyl.

$R_{25}$ ist als $C_3$-$C_8$ Alkenyl z.B. Allyl, 2-Butenyl, 2-Hexenyl oder 2-Octenyl, insbesondere Allyl.

$R_{26}$ ist als $C_1$-$C_{18}$ Alkyl z.B. Methyl, Aethyl, Propyl, Butyl, Hexyl, Octyl, Dodecyl oder Octadecyl. Bevorzugte Alkyl sind solche mit 1-12, insbesondere 1-8 C-Atomen, und vor allem solche mit 1-4 C-Atomen.

Stellt A ein Amin der Formel XVIII dar, so bedeuten dessen Substituenten $R_{27}$, $R_{28}$ und $R_{29}$ beispielsweise $C_1$-$C_{18}$ Alkyl, wie Methyl, Aethyl, iso-Propyl, sec-Butyl, n-Hexyl, n-Octyl, n-Decyl, n-Dodecyl oder n-Octadecyl, bevorzugt $C_1$-$C_8$ Alkyl.

Handelt es sich dabei um substituiertes Alkyl, so ist damit insbesondere $C_1$-$C_{18}$ Hydroxyalkyl gemeint, wie 1-Hydroxyäthyl, 1-Hydroxypropyl, 1-Hydroxy-1-methyläthyl, 3-Hydroxy-pentyl, oder 1-Hydroxy-2-methyl-äthyl, bevorzugt $C_1$-$C_8$ Hydroxyalkyl.

$R_{27}$ und $R_{28}$ können auch $C_5$-$C_{12}$ und insbesondere $C_5$-$C_6$ Cycloalkyl bedeuten, wobei es sich z.B. um Cyclopentyl, Cyclohexyl, 4-Methylcyclohexyl oder 4-tert.-Butylcyclohexyl handeln kann. $R_{27}$ kann auch die Bedeutung von gegebenenfalls substituiertem $C_6$-$C_{10}$ Aryl haben, wobei es sich z.B. um Phenyl, Tolyl, Xylyl, tert.-Butylphenyl oder Dodecylphenyl handeln kann. $R_{27}$ als $C_7$-$C_{20}$ Aralkyl ist z.B. Benzyl, 4-Methylbenzyl, 4-t.-Butylbenzyl oder 4-Dodecylbenzyl, insbesondere Benzyl.

Bedeuten $R_{27}$ und $R_{28}$ eine gegebenenfalls substituierte Aminoalkylgruppe so handelt es sich insbesondere um eine mit einer Piperidinylgruppe substituierte Aminoalkylgruppe, z.B. der Formel VIIb

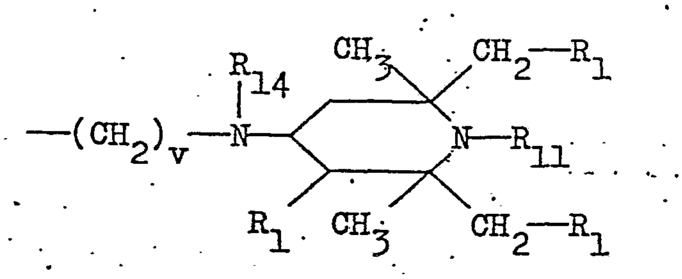

(VIIb) ,

worin $R_1$, $R_{11}$ und $R_{14}$ die oben angegebene Bedeutung haben und v eine ganze Zahl von 1 bis 8 ist.

Bedeuten $R_{27}$ und $R_{28}$ eine gegebenenfalls substituierte Piperidinylgruppe, so handelt es sich dabei vorzugsweise um eine solche der Formel VI.

$R_{27}$ und $R_{28}$ können zusammen mit dem N-Atom des Amins der Formel XVIII einen durch Alkylgruppen substituierten oder unsubstituierten Pyrrolidin-, Piperidin- oder Morpholinring bilden. Es kann sich in diesem Fall beispielsweise um einen 2,5-Dimethylpyrrolidin-, 4-Methylpiperidin-, 2,2,6,6-Tetramethylpiperidin-, 2,6-Dimethylmorpholin-, Pyrrolidin-, Piperidin- oder Morpholinring handeln, insbesondere um einen 2,2,6,6-Tetramethylpiperidinring.

m kann 0, 1 oder 2 sein, bevorzugt bedeutet m 0.

In seiner Bedeutung als Amin stellt also der Ligand A definitionsgemäss ein primäres, sekundäres oder tertiäres Amin dar, das in der Lage ist, mit den oben angeführten Metallchelaten einen Komplex zu bilden. Beispiele für solche Amine sind: n-Butylamin, n-Dodecylamin, β-Aethylhexylamin, Benzylamin, 4-Octylbenzylamin, Dibutylamin, Dicyclohexylamin, Dioctadecylamin, Morpholin, 2,2,6,6-Tetramethylpiperidin, N-Aethylanilin, Tri-n-octylamin, N,N-Dimethylanilin, N,N-Dimethyl-cyclohexylamin, N-Aethylpiperidin, N-Methylpyrrolidin, Dibenzylpropylamin, N-Benzyl-2,5-dimethylpyrrolidin, 4-Dimethylamino-2,2,6,6-

tetramethylpiperidin, 4-Dimethylamino-1,2,2,6,6-pentamethylpiperidin, N,N'-Methyl-N,N'-(1,2,2,6,6-pentamethyl-4-piperidyl)-äthylendiamin, Hydroxyäthylamin, Di-(hydroxyäthyl)-amin, Tri-(hydroxyäthyl)-amin, Tri-(2-hydroxy-propyl)-amin, N-Phenyl-N,N-di-(hydroxyäthyl)-amin oder Hydroxypropyl-amin.

Die erfindungsgemässen Komplexe der Formel I enthalten pro Mol Metall $M^{q\oplus}$ 0 bis 2 Mole des Aminliganden A, welcher ganz oder teilweise durch Wasser ersetzt sein kann. Der in der Formel I mit m ausgedrückte Molanteil setzt also aus der Summe von m' Molen Aminligand und m'' Molen Wasser zusammen, wobei die durch m' und m'' ausgedrückten Molanteile keine ganze Zahl zu sein brauchen. Bevorzugte Komplexe sind jene mit geringem Wassergehalt, da sich diese besser in unpolaren Polymeren lösen als stärker hydratisierte Komplexe. m' nimmt damit annähernd den Wert von m an.

Ein aminischer Ligand A ist nur dann notwendig, wenn die Koordinationszahl des Metallzentrums nicht durch den Chelatbildner L allein erfüllt wird.

Bevorzugte Verbindungen der Formel I sind solche, worin

| | |
|---|---|
| $M^{q\oplus}$ | $Mg^{2+}$, $Ca^{2+}$, $Sr^{2+}$, $Ba^{2+}$, $Zn^{2+}$, $Cd^{2+}$, $Al^{3+}$, $Sn^{2+}$, $Cr^{3+}$, $Co^{2+}$, $Ni^{2+}$, $VO^{2+}$, $MoO^{2+}$, $(R^*)_2Sn^{2+}$ oder $(CH_2CH_2COOR^*)_2Sn^{2+}$ bedeutet, worin |
| R* | $C_1$-$C_8$ Alkyl ist, und |
| q | 2 oder 3 ist, und |
| r | 1 oder 2 ist, wobei der Quotient q/r 1, 1.5, 2 oder 3 bedeutet, und |
| L | eine Gruppe der Formel II ist, worin |

BAD ORIGINAL

| | |
|---|---|
| $R_1$ | Wasserstoff oder Methyl ist, und |
| $Z_1$ | Wasserstoff, $C_1$-$C_8$ Alkyl, $C_3$-$C_4$ Alkenyl, $C_3$ Alkinyl, $C_2$-$C_{10}$ Alkoxyalkyl, $C_7$-$C_8$ Aralkyl, Acetyl oder eine der Gruppen $-CH_2COOR_2$ oder $-CH_2-CH(R_3)-OR_4$, oder einen Rest $-(CH_2)_4-R_5$, $-CH_2-CH=CH-CH_2-R_5$, 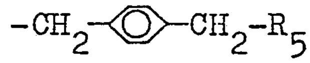, 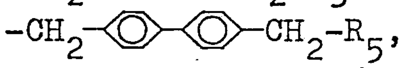, oder eine Gruppe der Formel III bedeutet, wobei |
| $R_2$ | $C_1$-$C_4$ Alkyl, $C_3$-$C_4$ Alkenyl, Phenyl, $C_7$-$C_8$ Aralkyl oder Cyclohexyl ist, und |
| $R_3$ | Wasserstoff oder Methyl ist, |
| $R_4$ | eine aliphatische Acylgruppe mit 1-12 C-Atomen ist, und |
| $R_5$ | eine Gruppe der Formel IV ist, worin $Z_2$ und $Z_3$ die unten angegebene Bedeutung haben und $R_1$ Waswerstoff ist, und |
| $R_6$ | eine Gruppe der Formel V bedeutet, worin $R_9$ Wasserstoff oder Methyl ist, und |
| $R_7$ und $R_8$ | unabhängig voneinander Wasserstoff oder Methyl sind, und |
| a | 0, 1 oder 2, und |
| b | 0 oder 1, und |
| c | 0 oder, falls a und/oder b verschieden von 0 sind, auch 1 ist, und |
| X | -O- ist, und |
| Y | -O- oder $-\overset{|}{N}-R_{10}$ ist, wobei |
| $R_{10}$ | Wasserstoff oder Methyl oder falls a, b und c 1, $R_7$ Wasserstoff und Y -O- sind, auch eine Gruppe $-CH_2-CH_2-OR_6$, worin $R_6$ die oben angegebene Bedeutung hat, bedeutet, und |
| $Z_2$ | Wasserstoff, $C_2$-$C_{12}$ aliphatischer Acyloxy, gegebenenfalls mit Methyl oder Methoxy mono- oder disubstituiertes Benzoyloxy, oder eine gegebenen- |

falls $C_1$-$C_4$ N-alkylsubstituierte $C_2$-$C_{12}$ Acylamidogruppe ist, oder eine der Gruppen-$R_{12}$, $-(CH_2)_d-R_{12}$, $-CH_2-$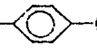$-CH_2-R_{12}$ oder $-CH_2-$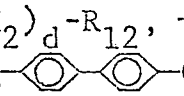$-CH_2-R_{12}$ ist, worin d 2 bis 6 ist, und

$R_{12}$ eine Gruppe der Formel VII ist, worin $R_1$ und $Z_1$ die oben angegebene Bedeutung haben, oder

$Z_2$ ferner eine Gruppe der Formel VIII bedeutet, worin

X' -O- oder $-\overset{|}{N}-R_{14}$ ist, wobei

$R_{14}$ Wasserstoff oder $C_1$-$C_4$ Alkyl ist, und

$R_{13}$ $-(CH_2)_e-$ oder eine Gruppe

$$-\overset{R_{15}}{\overset{|}{\underset{\underset{R_{16}}{|}}{C}}}-$$

ist, wobei

e 0 bis 8 ist, und

$R_{15}$ und $R_{16}$ unabhängig voneinander Wasserstoff, $C_1$-$C_4$ Alkyl, Allyl, Benzyl, Cyanomethyl, Cyanoäthyl oder eine Gruppe $-CH_2COOR_{17}$, wobei

$R_{17}$ Methyl oder Aethyl ist, bedeuten, oder

$Z_2$ eine Gruppe der Formeln IX, X oder XI ist, worin

$R_6$ und $R_7$ die oben angegebene Bedeutung haben,

$R_{18}$ Wasserstoff bedeutet,

$R_{19}$ Wasserstoff, $C_1$-$C_4$ Alkyl, Allyl oder Benzyl ist,

$R_{20}$ $C_1$-$C_4$ Alkyl oder eine Gruppe der Formel V mit oben angegebener Bedeutung ist, und

$R_{21}$ $C_2$-$C_6$ Alkylen, Cyclohexyliden oder Phenylen ist oder

$Z_2$ ferner eine Gruppe der Formel XIIa

BAD ORIGINAL

$$-(X_1)_f-(CH_2)_a-\overset{\displaystyle R_7}{\overset{|}{(CH)}}_b-(Y_1)_c-R_6 \qquad (XII\,a)$$

ist, worin

a, b, c, $R_6$ und $R_7$ oben angegebene Bedeutung haben, und

f — 0 oder 1 ist, und

$X_1$ — -O- oder $-\overset{|}{N}-R^*_{10}$ bedeutet, worin

$R^*_{10}$ — Wasserstoff, $C_1$-$C_{12}$ Alkyl, Cyclohexyl, $C_3$-$C_{14}$ Alkoxyalkyl, $C_7$-$C_8$ Aralkyl, gegebenenfalls mit $C_1$-$C_3$ Alkyl und/oder $C_1$-$C_8$ Alkoxy mono- oder disubstituiertes Phenyl oder falls a, b und c 1, $R_7$ Wasserstoff und Y -O- sind, auch eine Gruppe $-CH_2-CH_2-OR_6$, worin $R_6$ die oben angegebene Bedeutung hat, ist, und $R^*_{10}$ ferner eine Gruppe VI bedeutet, worin

$R_1$ — die oben angegebene Bedeutung hat,

$R_{11}$ — Wasserstoff, Methyl oder Allyl ist, und

$Y_1$ — -O- oder $-\overset{|}{N}-R^{**}_{10}$ bedeutet, worin

$R^{**}_{10}$ — Wasserstoff, $C_1$-$C_8$ Alkyl oder Phenyl ist, oder

$Z_2$ — ferner eine der Gruppen XIII oder XIV ist, worin

$R_6$ — die oben angegebene Bedeutung hat, und

$R_{22}$ und $R_{23}$ — unabhängig voneinander Wasserstoff, Methyl oder Aethyl sind, und

$Z_3$ — Wasserstoff oder Cyano ist, oder

$Z_2$ und $Z_3$ — zusammen eine der Formeln XV, XVI oder XVII bedeuten, worin $R_6$ die oben angegebene Bedeutung hat, und

$R_{24}$ — Wasserstoff, Methyl oder Aethyl ist und

$R_{25}$ — Wasserstoff, $C_1$-$C_{12}$ Alkyl, $C_3$-$C_8$ Alkenyl, eine Gruppe $-CH_2COOR_{26}$ oder $-CH_2-CH(R_8)-OR_6$ bedeutet, wobei $R_6$ und $R_8$ die oben angegebene Bedeutung haben, und

$R_{26}$ $C_1$-$C_{12}$ Alkyl ist, und
m 0, 1 oder 2 ist, und
A $H_2O$ oder ein Amin der Formel XVIII ist, worin
$R_{27}$ gegebenenfalls mit -OH substituiertes $C_1$-$C_{18}$ Alkyl, $C_5$-$C_{12}$ Cycloalkyl, gegebenenfalls mit $C_1$-$C_{12}$ Alkyl substituiertes $C_6$-$C_{10}$ Aryl, $C_7$-$C_{20}$ Aralkyl, eine gegebenenfalls substituierte Aminoalkylgruppe mit 1-8 C-Atomen im Alkylteil oder Piperidinylgruppe ist, und
$R_{28}$ Wasserstoff, oder gegebenenfalls mit -OH substituiertes $C_1$-$C_{18}$ Alkyl, $C_5$-$C_{12}$ Cycloalkyl oder eine gegebenenfalls substituierte Aminoalkylgruppe mit 1-8 C-Atomen im Alkylteil oder Piperidinylgruppe bedeutet, oder
$R_{27}$ und $R_{28}$ zusammen mit dem N-Atom einen durch Alkylgruppen substituierten oder unsubstituierten Pyrrolidin-, Piperidin- oder Morpholinring bilden, und
$R_{29}$ Wasserstoff oder gegebenenfalls mit -OH substituiertes $C_1$-$C_{18}$ Alkyl bedeutet.

Besonders bevorzugt sind Verbindungen der Formel I, worin
$M^{q\oplus}$ $Ca^{2+}$, $Mg^{2+}$, $Zn^{2+}$, $Co^{2+}$, $Ni^{2+}$ oder $Al^{3+}$ ist, und
q 2 oder 3 ist, und
r 1 oder 2 ist, wobei der Quotient q/r 1, 1.5, 2 oder 3 ist, und
L eine Gruppe der Formel II ist, worin
$R_1$ Wasserstoff oder Methyl ist, und
$Z_1$ Wasserstoff, $C_1$-$C_4$ Alkyl, Allyl, $C_2$-$C_6$ Alkoxyalkyl, Benzyl, Acetyl oder eine der Gruppen $-CH_2COOR_2$ oder $-CH_2-CH(R_3)-OR_4$ oder eine Gruppe der Formel III bedeutet, wobei
$R_2$ $C_1$-$C_4$ Alkyl ist, und

$R_3$ Wasserstoff ist, und

$R_4$ eine aliphatische Acylgruppe mit 1-8 C-Atomen ist, und

$R_6$ eine Gruppe der Formel V bedeutet, worin $R_9$ Wasserstoff ist, und

$R_7$ Wasserstoff ist, und

$R_8$ Wasserstoff oder Methyl bedeutet, und

a 0, 1 oder 2 ist, und

b 0 oder 1 ist, und

c 0 oder, falls a und/oder b von 0 verschieden sind, auch 1 ist, und

X -O- ist, und

Y -O- oder $-\overset{|}{N}-R_{10}$ bedeutet, wobei

$R_{10}$ Wasserstoff oder Methyl oder, falls a, b und c 1, und Y -O- sind auch eine Gruppe $-CH_2-CH_2-OR_6$, worin $R_6$ die oben angegebene Bedeutung hat, ist, und

$Z_2$ Wasserstoff, $C_2$-$C_7$ aliphatisches Acyloxy, gegebenenfalls mit Methyl oder Methoxy monosubstituiertes Benzoyloxy, oder eine gegebenenfalls mit Methyl N-substituierte $C_2$-$C_7$ Acylamidogruppe ist oder eine der Gruppen $-(CH_2)_d-R_{12}$, 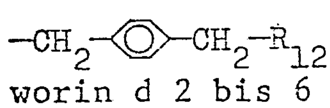 oder 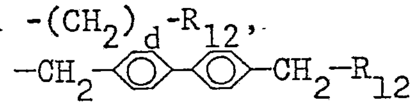 ist, worin d 2 bis 6 ist, und

$R_{12}$ eine Gruppe der Formel VII ist, worin $R_1$ und $Z_1$ die oben angegebene Bedeutung haben, oder

$Z_2$ ferner eine Gruppe der Formel VIII bedeutet, worin

X' -O- oder $-\overset{|}{N}-R_{14}$ ist, wobei

$R_{14}$ Wasserstoff oder Methyl ist, und

$R_{13}$ $-(CH_2)_e-$ oder eine Gruppe $-\overset{R_{15}}{\underset{R_{16}}{C}}-$ ist, wobei

e 0 bis 8 ist, und

$R_{15}$ und $R_{16}$ unabhängig voneinander Wasserstoff, $C_1$-$C_4$ Alkyl, Allyl, Benzyl oder Cyanomethyl sind, oder

$Z_2$ ferner eine Gruppe der Formel XI ist, worin

$R_{20}$ eine Gruppe derFormel V mit oben angegebener Bedeutung ist, und

$R_{21}$ $C_2$-$C_6$ Alkylen oder Phenylen bedeutet, oder

$Z_2$ ferner eine Gruppe der Formel XIIa ist, worin a, b, c, $R_6$ und $R_7$ oben angegebene Bedeutung haben, und

f 0 oder 1 ist, und

$X_1$ -O- oder $-\overset{|}{N}-R_{10}^{*}$ bedeutet, worin

$R_{10}^{*}$ Wasserstoff, $C_1$-$C_8$ Alkyl, Cyclohexyl, $C_3$-$C_8$ Alkoxyalkyl, $C_7$-$C_8$ Aralkyl, gegebenenfalls mit $C_1$-$C_3$ Alkyl und/oder $C_1$-$C_2$ Alkoxy monosubstituiertes Phenyl oder falls a, b und c 1 und $Y_1$ -O- sind, auch eine Gruppe $-CH_2-CH_2-OR_6$, worin $R_6$ die oben angegebene Bedeutung hat, ist, und $R_{10}^{*}$ ferner eine Gruppe VI bedeutet, worin

$R_1$ die oben angegebene Bedeutung hat, und

$R_{11}$ Wasserstoff oder Methyl ist, und

$Y_1$ -O- oder $-\overset{|}{N}-R_{10}^{**}$ bedeutet, worin

$R_{10}^{**}$ Wasserstoff, $C_1$-$C_4$ Alkyl oder Phenyl ist, oder

$Z_2$ ferner eine der Gruppen XIII oder XIV ist, worin

$R_6$ die oben angegebene Bedeutung hat, und

$R_{22}$ und $R_{23}$ unabhängig voneinander Wasserstoff, Methyl oder Aethyl sind, und

$Z_3$ Wasserstoff ist, oder

$Z_2$ und $Z_3$ zusammen eine der Formeln XV, XVI oder XVII bedeuten, worin $R_6$ die oben angegebene Bedeutung hat, und

$R_{24}$ Wasserstoff oder Methyl ist, und

$R_{25}$ $C_1$-$C_8$ Alkyl, Allyl, oder eine der Gruppen

$-CH_2-COOR_{26}$ oder $-CH_2-CH(R_8)-OR_6$ bedeutet, worin $R_6$ und $R_8$ die oben angegebene Bedeutung haben, und

$R_{26}$ $C_1$-$C_8$ Alkyl ist, und

m 0, 1 oder 2 ist, und

A $H_2O$ oder ein Amin der Formel XVIII ist, worin

$R_{27}$ und $R_{28}$ unabhängig voneinander $C_1$-$C_8$ Alkyl, $C_1$-$C_6$ Hydroxyalkyl oder eine substituierte Aminoalkylgruppe der Formel VIIb

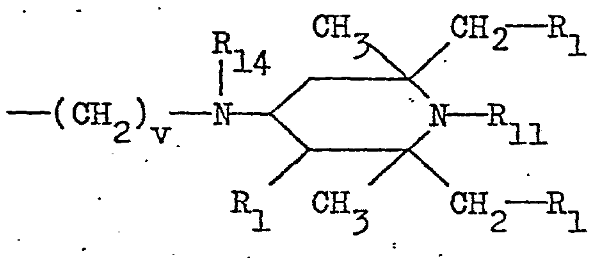

(VIIb)

ist, worin $R_1$, $R_{11}$ und $R_{14}$ die oben angegebene Bedeutung haben und v 1 bis 8 ist, oder

$R_{27}$ und $R_{28}$ zusammen mit dem N-Atom eine Piperidingruppe bilden, und

$R_{29}$ Wasserstoff, $C_1$-$C_8$ Alkyl oder $C_1$-$C_6$ Hydroxyalkyl ist.

Besonderes Interesse gilt Verbindungen der Formel I, worin

$M^{q\oplus}$ $Ca^{2+}$, $Mg^{2+}$, $Zn^{2+}$, $Co^{2+}$, $Ni^{2+}$ oder $Al^{3+}$ ist, und

q 2 oder 3 ist, und

r 1 oder 2 ist, wobei der Quotient q/r 1, 1.5, 2 oder 3 ist, und

L eine Gruppe der Formel II ist, worin

$R_1$ Wasserstoff ist, und

$Z_1$ Wasserstoff, Methyl, Acetyl oder eine Gruppe der Formel III bedeutet, wobei

$R_6$ eine Gruppe der Formel V bedeutet, worin $R_9$ Was-

serstoff ist, und

$R_7$ Wasserstoff und

$R_8$ Wasserstoff oder Methyl ist, und

a 0, 1 oder 2 ist, und

b 0 oder 1 und

c 0 oder, falls a und/oder b von 0 verschieden sind, auch 1 ist,

X -O- ist, und

Y $-\overset{|}{N}-R_{10}$ bedeutet, wobei

$R_{10}$ Wasserstoff ist, und

$Z_2$ Wasserstoff, $C_2$-$C_7$ aliphatisches Acyloxy, Benzoyloxy, eine gegebenenfalls mit Methyl N-substituierte Acylamidogruppe ist, oder eine der Gruppen $-(CH_2)_d-R_{12}$, 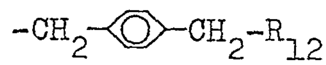 oder 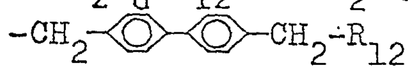 ist, worin d 2 bis 6 ist, und

$R_{12}$ eine Gruppe der Formel VII ist, worin $R_1$ und $Z_1$ die oben angegebene Bedeutung haben, oder

$Z_2$ ferner eine Gruppe der Formel VIII ist, worin

X' -O- oder $-\overset{|}{N}-R_{14}$ bedeutet, wobei

$R_{14}$ Wasserstoff oder Methyl ist, und

$R_{13}$ $-(CH_2)_e-$ oder eine Gruppe $-\underset{R_{16}}{\overset{R_{15}}{C}}-$ ist, wobei

e 0 bis 8 ist, und

$R_{15}$ und $R_{16}$ unabhängig voneinander Wasserstoff, $C_1$-$C_4$ Alkyl, Allyl oder Benzyl sind, oder

$Z_2$ ferner eine Gruppe der Formel XI ist, worin

$R_{20}$ eine Gruppe der Formel V mit oben angegebener Bedeutung ist, und

$R_{21}$ $C_2$-$C_6$ Alkylen ist, oder

$Z_2$ ferner eine Gruppe der Formel XIIa ist, worin a, b, c, $R_6$ und $R_8$ oben angegebene Bedeutung haben, und

| | |
|---|---|
| f | 0 oder 1 ist, und |
| $X_1$ | -O- oder $-\overset{\vert}{N}-R^*_{10}$ bedeutet, worin |
| $R^*_{10}$ | Wasserstoff, $C_1$-$C_8$ Alkyl, $C_3$-$C_6$ Alkoxyalkyl, $C_7$-$C_8$ Aralkyl, gegebenenfalls mit Methyl, Metho: oder Aethoxy monosubstituiertes Phenyl oder ein( Gruppe VI bedeutet, worin |
| $R_1$ | die oben angegebene Bedeutung hat, und |
| $R_{11}$ | Wasserstoff oder Methyl ist, und |
| $Y_1$ | -O- ist, oder |
| $Z_2$ | ferner eine der Gruppen XIII oder XIV ist, worir |
| $R_6$ | die oben angegebene Bedeutung hat, und |
| $R_{22}$ und $R_{23}$ | unabhängig voneinander Wasserstoff oder Methyl sind, und |
| $Z_3$ | Wasserstoff ist, oder |
| $Z_2$ und $Z_3$ | zusammen eine der Formeln XV, XVI oder XVII bedeuten, worin $R_6$ die oben angegebene Bedeutung hat, und |
| $R_{24}$ | Wasserstoff ist, und |
| $R_{25}$ | $C_1$-$C_8$ Alkyl, oder eine der Gruppen $-CH_2COOR_{26}$ oder $-CH_2-CH(R_8)-OR_6$ bedeutet, worin $R_6$ und $R_8$ oben angegebene Bedeutung haben, und |
| $R_{26}$ | $C_1$-$C_4$ Alkyl ist, und |
| m | 0, 1 oder 2 ist, und |
| A | $H_2O$ oder ein Amin der Formel XVIII ist, worin |
| $R_{27}$ und $R_{28}$ | unabhängig voneinander $C_1$-$C_8$ Alkyl, $C_1$-$C_6$ Hydroxyalkyl oder eine Gruppe VIIb sind, worin $R_1$, $R_{11}$ und $R_{14}$ die angegebene Bedeutung haben, und v 1 bis 8 ist, oder |
| $R_{27}$ und $R_{28}$ | zusammen mit dem N-Atom eine Piperidingruppe bi: den, und |
| $R_{29}$ | Wasserstoff, $C_1$-$C_8$ Alkyl oder $C_1$-$C_6$ Hydroxyalky: ist. |

Beispiele für Verbindungen der Formel I sind Metallkomplexe eines Metalls $M^{q\oplus}$ mit einem der folgenden Liganden:

2,3,6-Trimethyl-2,6-diäthyl-4-(1',3'-dioxobutyl-oxy)-piperidin

2,2,6,6-Tetramethyl-4-(1',3'-dioxopentyloxy)-piperidin

1-{2"-[N-n-Butyl-N-(1',3'-dioxobutyl)]-amino-2"-methyläthyl}-2,2,6,6-tetramethyl-4-heptanoyloxy-piperidin

1-[(2'-Phenyl-3',6'-dioxa-5'-methyl-7',9'-dioxo)-decyl]-2,2,6,6-tetramethyl-piperidin

N,N-Dimethyl-N,N'-bis-[1-(3'-oxa-4',6'-dioxo)-heptyl-2,2,6,6-tetramethyl-piperidin-4-yl]-hexamethylendiamin

Bis-[1-(3'-oxa-4',6'-dioxo)-heptyl-2,2,6,6-tetramethyl-piperidin-4-yl]-p-xyliden

Bis-[1-(3'-oxa-4',6'-dioxo)-heptyl-2,2,6,6-tetramethyl-piperidin-4-yl]-adipat

Bis-[1-(3'-oxa-4',6'-dioxo)-heptyl-2,2,6,6-tetramethyl-piperidin-4-yl]-n-butyl-malonat

Bis-[1-(3'-oxa-4',6'-dioxo)-heptyl-2,2,6,6-tetramethyl-piperidin-4-yl]-methyl-carbäthoxymethyl-malonat

3-n-Octyl-7,7,9,9-tetramethyl-8-[(3'-oxa-4',6'-dioxo)-heptyl]-1,3,8-triazaspiro-[4,5]-decan-2,4-dion

1,2,2,6,6-Pentamethyl-4-[1',4',8'-trioxa-9',11'-dioxo-6'-(1",3"-dioxobutyloxy)]-piperidin

2,2,6,6-Tetramethyl-4-[4'-(1",3"-dioxobutyloxy)-3'-t.butyl-phenyl]-piperidin

2,2,6,6-Tetramethyl-4-[3',5'-dimethyl-4'-(1",3"-dioxo-butyloxy)-cyclohexyl]-piperidin

3-Aethyl-3-(1',3'-dioxo-butyloxy-methyl)-8,8,10,10-tetramethyl-1,5-dioxa-9-aza-spiro[5,5]-undecan

3-(2'-Methyl-3'-oxa-4',6'-dioxo-heptyl)-7,7,9,9-tetramethyl-1,3,8-triazaspiro[4,5]-decan-2,4-dion

Bis-(2,2,6,6-tetramethyl-piperidin-4-yl)-[2'-methoxymethyl-3'-oxa-4',6'-dioxo-heptyl]-malonat

Bis-(2,2,6,6-tetramethyl-piperidin-4-yl)-methyl-[N-benzyl-N-(1',3'-dioxo-butyl)-aminopropyl]-malonat

2,2,6,6-Tetramethyl-4-cyano-4-(1',3'-dioxo-butyloxy)-piperidin.

Die hier aufgelisteten Liganden können nach einem der unten geschilderten Verfahren in Metallkomplexe mit einem Metall $M^{q\oplus}$ übergeführt werden.

Die erfindungsgemässen Verbindungen zeichnen sich dadurch aus, dass die darin komplexierten Liganden L mindestens einen Rest $R_6$ mit oben beschriebener Bedeutung enthalten. Die zur Synthese verwendeten Liganden der Formel II sind, mit Ausnahme des Acetessig-(2,2,6,6-tetramethyl-4-piperidinyl)-esters, welcher ebenfalls unter die allgemeine Formel II fällt, neu und stellen daher auch einen Bestandteil der vorliegenden Erfindung dar. Die Liganden der Formel II können auf bekannte Weise aus den analogen Verbindungen der Formel II', worin in den Resten $Z_1$ und $Z_2$ der Substituent $R_6$ durch Wasserstoff ersetzt ist, und alle übrigen Symbole die in der Formel II angegebene Bedeutung haben, hergestellt werden. Insbesondere wird dabei so vorgegangen, dass die entsprechenden Hydroxy-

bzw. Aminoderivate direkt oder in einem Lösungsmittel mit einem der Formel V entsprechenden Deketen bzw. einem Diketen-Homologen umgesetzt werden, wobei sich meist ein Zusatz eines Katalysators erübrigt (vgl. Houben-Weyl "Methoden der organischen Chemie" 4. Auflage, Band 7/4 Seite 226ff). Dabei wird das Diketen vorzugsweise in stöchiometrisch äquivalenter Menge oder in einem bis 10%-igen Ueberschuss eingesetzt. Als Lösungsmittel für diese Reaktionen eignen sich insbesondere Benzol, Toluol, Dioxan, Dichloräthan, Pyridin oder Dimethylformamid. Andererseits können diese Verbindungen aber aus den durch obige Definition beschriebenen Zwischenprodukten der Formel II' und $\beta$-Ketocarbonsäureestern durch Umesterung bzw. Aminolyse von Carbonsäureestern in an sich bekannter Weise gewonnen werden.

Jene Zwischenprodukte (der allgemeinen Formel II'), in denen geminale Hydroxygruppen auftreten, können aus 2,2,6,6-Tetraalkylpiperidinderivaten, die primäre oder sekundäre Aminogruppen oder primäre Hydroxygruppen enthalten, und 2,3-Epoxy-1-propanol nach den bekannten Methoden der Epoxydumsetzungen erhalten werden.

Die Ausgangsstoffe der Formel II' sind bekannt oder können, sofern sie neu sind, nach an sich bekannten Methoden und in Analogie zu bekannten Verbindungen hergestellt werden. So stellen beispielsweise 4-Aminopiperidin-Derivate wichtige Ausgangsprodukte dar, die sich nach den in der DT-OS 2 040 975 bzw. DT-OS 2 349 962 beschriebenen Verfahren herstellen lassen. In analoger Weise lassen sich jene Piperidinderivate herstellen, die an der 4-Aminogruppe durch eine Alkylaminogruppe substituiert sind, indem man von einem Piperidin-4-on-Derivat ausgeht und dieses mit einem monoalkylierten Diamin umsetzt.

Die Piperidinderivate, die in 4-Stellung eine Alkoxyaminogruppe (insbesondere die Oxypropylaminogruppe) enthalten, werden aus den entsprechenden 4-Hydroxypiperidi derivaten hergestellt, die man nach an sich bekannten Methoden an Acrylnitril anlagert, anschliessend wird die Cyanogruppe zum entsprechenden primären Amin hydriert. Weitere wichtige Ausgangsprodukte zur Herstellung von Verbindungen nach Formel I sind jene Verbindungen, die in 4-Stellung des Piperidin-Ringes zugleich eine Cyano- und eine Hydroxygruppe(Cyanhydrine) oder zugleich eine Cyanogruppe und eine Aminogruppe tragen. Ihre Herstellung wird

im US-Patent 3,513,170 beschrieben. Durch Hydrierung dieser Nitrile erhält man dann in bekannter Weise die entsprechenden 4-Methylamino-Derivate. α-Aminocarbonsäureester, deren α-Kohlenstoffatom in den in Frage kommenden Piperidinderivaten in 4-Stellung steht, sind ebenfalls Ausgangsprodukte zur Herstellung von beanspruchten Verbindungen der Formel I.

Die freien Säuren können aus den entsprechenden Piperidin-4-on-derivaten nach der Methode von H.T. Bucherer (J. prakt. Chem. 140, 291 (1934)) über Hydantoine als Zwischenprodukte und anschliessende alkalische Verseifung hergestellt werden. Die Ueberführung in die entsprechenden Ester erfolgt in an sich bekannter Weise mit Hilfe eines sauren Katalysators.

Die am Stickstoff in 1-Stellung substituierten Piperidin-4-on-derivate können, wie das im Brit.-Patent 1 337 600 beschrieben ist, dadurch erhalten werden, dass man die Ketogruppe durch Ueberführung in ein Ketal schützt. Nach der Einführung des Substituenten in 1-Stellung nach den üblichen Methoden wird die Schutzgruppe in bekannter Weise wieder abgespaltet.

Die Herstellung der Metallkomplexe der Verbindungen der Formel I erfolgt in an sich bekannter Weise, z.B. dadurch, dass die β-Ketoester bzw. β-Ketoamide der Formel II in einem nicht-wässrigen Lösungsmittel, vorzugsweise einem niederen Alkohol (insbesondere Aethanol) aufgelöst und eine den enolisierbaren Ketogruppen äquivalente Menge eines Alkalialkoholates zugefügt werden. Darauf werden soviele Mole eines gelösten Metallsalzes langsam zugefügt, dass das molare Verhältnis zwischen dem Metallion

der Wertigkeit q+ und den neutralisierten Enolatgruppen des Ligandmoleküls r/q beträgt. Als Lösungsmittel für das Metallsalz kommt vor allem das anfangs verwendete oder ein damit mischbares in Frage. Das Reaktionsgemisch wird anschliessend bei einer Temperatur zwischen 30-100°C ca. 1 Stunde gerührt. Als Metallsalze kommen vorzugsweise jene in Frage, deren Anionen mit den Alkaliionen in den gewählten Lösungsmitteln schwerlösliche Fällungen ergeben. Dies erfolgt meist bei der Zugabe des Metallsalzes oder beim anschliessenden Rühren oder Erhitzen des Reaktionsgemisches. In diesen Fällen können die Alkalisalze durch Filtration abgetrennt werden. Andernfalls können sie oft nach Verdampfen des Lösungsmittels oder durch Extraktion der Metallverbindung mit einem unpolaren Lösungsmittel abgetrennt werden. - Als Metallsalze kommen insbesondere Chloride, wie Nickelchlorid, Cobalt(II)chlorid oder Dibutylzinndichlorid, in Frage. Verwendet man nicht wasserfreie Salze oder enthalten die verwendeten Lösungsmittel Wasser, kann dieses in den sich bildenden Komplex als solches oder aber in deprotonierter Form als Hydroxylion eingebaut werden. In diesen Fällen enthalten die Endprodukte oft noch das Alkalimetall, das als Alkoholat zur Bildung der Enolatgruppen verwendet wurde. Verwendet man als Metallsalz ein Salz einer Carbonsäure (z.B. das Nickelsalz der 2-Aethylcapronsäure), lässt sich das Carboxylation oft nicht oder nur teilweise als Alkalisalz abtrennen, weil es als weiteres Ligandanion neben den Enolationen in den Komplex eingebaut wird.

Statt anorganischen Metallsalzen und Metallcarboxylaten kommen als Umsetzungskomponenten zur Bildung von Metallkomplexen der Verbindung der Formel I auch Metallalkoxyde (wie z.B. Aluminiumtriisopropylat) in Frage. In diesen Fällen verzichtet man auf die Neutralisation des β-Ketoesters bzw. β-Ketoamides mit einem Alkalialkoholat.

Enthalten die Metallkomplexe der Verbindungen der Formel I ein Amin als weiteren Liganden, so kann dieses schon vor der Zugabe des Alkalialkoholes zugefügt werden oder man kann den in der oben beschriebenen Weise erhaltenen Metallkomplex in einem das Amin enthaltenden Lösungsmittel lösen und dieses dann durch Abdampfen wieder entfernen.

Geht man bei der Herstellung von Metallkomplexen der Verbindungen der Formel I in der beschriebenen Weise vor, entstehen oft Gemische verschiedener Komplexe, vor allem solche in denen das Verhältnis des Metallions zum Liganden L grösser als r/q ist. Sie können ebenso wie die einheitlichen Verbindungen dieser Formel als wirksame Stabilisatoren verwendet werden.

Die Verbindungen der Formel I können gemäss der vorliegenden Erfindung als Stabilisatoren für Kunststoffe gegen deren Schädigung durch Einwirkung von Sauerstoff, Wärme und Licht verwendet werden. Beispiele für solche Kunststoffe sind die in der DT-OS 2 456 864 auf den Seiten 12-14 aufgeführten Polymeren.

Von besonderer Bedeutung ist die Stabilisierung von Polyolefinen, Styrolpolymerisaten, Polyamiden und von Polyurethanen, für die sich die Verbindungen der Formel I hervorragend eignen. Beispiele hierfür sind Polyäthylen hoher und niedriger Dichte, Polypropylen, Aethylen-Propylen-Copolymerisate, Polystyrol, Styrol-Butadien-Acrylnitril-Tercopolymerisate, Mischungen von Polyolefinen oder von Styrolpolymerisaten, Polyurethane auf Polyäther- oder Polyesterbasis in Form von Lacken, Fäden, Filmen, Elastomeren oder Schaumstoffen.

Die Stabilisatoren werden den Kunststoffen in einer Konzentration von 0,01 bis 5 Gew.-%, berechnet auf das zu stabilisierende Material, zugesetzt. Vorzugsweise werden 0,03 bis 1,5, besonders bevorzugt 0,15 bis 0,6 Gew.-% der Verbindungen, berechnet auf das zu stabilisierende Material, in dieses eingearbeitet.

Die Einarbeitung kann nach der Polymerisation erfolgen, beispielsweise durch Einmischen der Verbindungen und gegebenenfalls weiterer Additive in die Schmelze nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels.

Die neuen Verbindungen können auch in Form eines Masterbatches, der diese Verbindungen beispielsweise in einer Konzentration von 2,5 bis 25 Gew.-% enthält, den zu stabilisierenden Kunststoffen zugesetzt werden.

Im Falle von vernetztem Polyäthylen werden die Verbindungen vor der Vernetzung beigefügt.

Die Erfindung betrifft daher auch die durch Zusatz von 0,01 bis 5 Gew.-% einer Verbindung der Formel I stabilisierten Kunststoffe, die gegebenenfalls noch andere bekannte und übliche Zusätze enthalten können. Die so stabilisierten Kunststoffe können in verschiedenster Form angewendet werden z.B. als Folien, Fasern, Bändchen, Profile oder als Bindemittel für Lacke, Klebemittel oder Kitte.

Als weitere Additive, mit denen zusammen die erfindungsgemäss verwendbaren Stabilisatoren eingesetzt werden können, sind beispielsweise zu nennen:

Antioxydantien, wie einfache 2,6-Dialkylphenole, Derivate von alkylierten Hydrochinonen, hydroxylierte Thiodiphenyläther, Alkyliden-bisphenole, O-, N- und S-Benzylverbindungen, hydroxybenzylierte Malonester, Hydroxybenzyl-Aromaten, s-Triazinverbindungen, Amide der β-(3,5-Di-tert.-butyl-4-hydroxyphenyl)-propionsäure, Ester der β-(3,5-Di-tert.-butyl-4-hydroxyphenyl)-propionsäure, Ester der β-(5-tert.-Butyl-4-hydroxy-3-methylphenyl)-propionsäure, Ester der 3,5-Di-tert.-butyl-4-hydroxyphenylessigsäure, Acylaminophenole, Benzylphosphonate, Aminoarylderivate, UV-Absorber und Lichtschutzmittel, wie 2-(2'-Hydroxyphenyl)-benztriazole, 2,4-Bis-(2'-hydroxyphenyl)-6-alkyl-s-triazine, 2-Hydroxybenzophenone, 1,3-Bis-(2'-hydroxybenzoyl)-benzole, Ester von gegebenenfalls substituierten Benzoesäuren, Acrylate, des weiteren Nickelverbindungen, sterisch gehinderte Amine, Oxalsäurediamide, Metalldesaktivatoren, Phosphite, peroxidzerstörende Verbindungen, Polyamidstabilisatoren, basische Co-Stabilisatoren, PVC-Stabilisatoren, Nukleierungsmittel oder sonstige Zusätze wie z.B. Weichmacher, Gleitmittel, Emulgatoren, Füllstoffe, Russ, Asbest, Kaolin, Talk, Glasfasern, Pigmente, optische Aufheller, Flammschutzmittel, Antistatica.

Beispiele für weitere Additive, mit denen zusammen die erfindungsgemäss verwendbaren Stabilisatoren eingesetzt werden können, finden sich in der DT-OS 2 427 853 auf Seiten 18-24.

Die Herstellung und Verwendung der erfindungsgemässen Verbindungen wird in den folgenden Beispielen näher beschrieben. Die Temperaturangaben betreffen immer °C.

## Beispiele 1 bis 10

Die Herstellung der Verbindungen der Beispiele 1-10 erfolgte nach folgender allgemeinen Vorschrift (siehe Tabelle 1)

0,1 Mole der in der Kolonne 2 genannten Verbindungen wurden in 100 ml Benzol gelöst und die in Kolonne 3 genannte Menge Diketen-(β-methylen-β-propiolakton), die mit Benzol im Verhältnis 1:1 vermischt wurde, innerhalb von 30 Minuten zugetropft. Die Temperatur des Reaktionsgemisches betrug nicht über 60°. Anschliessend wurde das Gemisch 4 Stunden auf 60° erwärmt und darauf das Lösungsmittel abgedampft. Der Rückstand wurde nach der in Kolonne 4 beschriebenen Methode aufgearbeitet. Das in Kolonne 5 genannte Endprodukt zeigte die in Kolonne 6 genannten Eigenschaften.

Tabelle 1

| 1. Bei-spiel | 2. Ausgangsprodukt | 3. Anzahl Mole Diketen | 4. Aufarbeitung |
|---|---|---|---|
| 1 | 1-(2'-Hydroxyäthyl)-2,2,6,6-tetramethylpiperidin | 0,103 | Destillation |
| 2 | 1-(2'-Hydroxyäthyl)-2,2,6,6-tetramethyl-4-hydroxy-piperidin | 0,210 | Kurzwegdestillation im Hochvakuum |
| 3 | 2,2,6,6-Tetramethyl-4-hydroxy-piperidin | 0,103 | Destillation |
| 4 | 1,2,2,6,6-Pentamethyl-4-hydroxy-piperidin | 0,103 | Destillation |
| 5 | 2,2,6,6-Tetramethyl-4-(N-n-butyl-amino)-piperidin | 0,103 | Destillation |
| 6 | 1,2,2,6,6-Pentamethyl-N-(N-n-heptylamino)-piperidin | 0,103 | Destillation |
| 7 | 2,2,6,6-Tetramethyl-4-[N-n-heptyl-N-(2'-hydroxyäthyl)-amino]-piperidin | 0,103 | Kurzwegdestillation im Hochvakuum |
| 8 | 2-Hydroxymethyl-7,7,9,9-tetramethyl-1,4-dioxy-8-azaspiro[4.5]-decan | 0,103 | Destillation |
| 9 | N,N'-Bis-(2,2,6,6-tetramethyl-4-piperidyl)-äthylendiamin | 0,210 | Umkristallisation aus Aethylen-glycoldi-methyl-äther |
| 10 | 1-(2',3'-dihydroxypropyl)-2,2,6,6-tetramethyl-piperidin | 0,210 | Umkristallisation aus Leichtbenzin (Kp 110-130°) |

Fortsetzung
Tabelle 1

| Bei-spiel | 5. Endprodukt | 6. Eigenschaften |
|---|---|---|
| 1 | 1-[2'-(1",3"-Dioxobutyloxy)-äthyl]-2,2,6,6-tetramethylpiperidin | Kp 140-142°/1,5 mm Hg |
| 2 | 1-[2'-(1",3"-Dioxobutyloxy)-äthyl]-4-(1",3"-dioxobutyloxy)-2,2,6,6-tetramethylpiperidin | bei 55° und 0,01 mm Hg flüchtige Fraktion · Elementaranalyse: gef. C 61,78% H 8,35% N 3,74% ber. 61,77% 8,46% 3,79% |
| 3 | 2,2,6,6-Tetramethyl-4-(1',3'-dioxobutyloxy)-piperidin | Kp 126-127°/1,5 mm Hg |
| 4 | 1,2,2,6,6-Pentamethyl-4-(1',3'-dioxobutyloxy)-piperidin. | Kp 114-115°/0,7 mm Hg |
| 5 | 2,2,6,6-Tetramethyl-4-[N-n-butylamin-N-(1',3'-dioxobutyl]-piperidin | Kp 187°/0,8 mm Hg |
| 6 | 1,2,2,6,6-Pentamethyl-4- [N-n-heptylamino-N-(1',3'-dioxobutyl) ]-piperidin | Kp 180-181°/0,9 mm Hg |
| 7 | 2,2,6,6-Tetramethyl-4-N-n-heptyl-N-[2'-(1",3"-dioxobutyloxy)-äthyl] -piperidin | bei 75° und 0,01 mm Hg flüchtige Fraktion · Elementaranalyse gef. C 69,9% H 11,2% N 7,4% ber. 69,65% 11,18% 7,06% |
| 8 | 2-(1',3'-Dioxobutyloxymethyl)-7,7,9,9-tetramethyl-1,4-dioxa-8-aza-spiro[4.5]-decan | Kp 136°/0,3 mm Hg |
| 9 | N,N'-Bis-(1',3'-dioxobutyl)-N,N'-bis-(2,2,6,6-tetramethyl-4-piperidyl)-äthylendiamin | F. 171° |
| 10. | 1-[2',3'-Bis-(1",3"-dioxobutyloxy)-propyl]-2,2,6,6-tetramethylpiperidin | F. 78-80° |

Fortsetzung Tabelle 1

| 1. Beispiel | 2. Ausgangsprodukt | 3. Anzahl Mole Diketen | 4. Aufarbeitung | 5. Endprodukt | 6. Eigenschaften |
|---|---|---|---|---|---|
| 11 | 1-(2'-Hydroxypropyl)-2,2,6,6-tetramethylpiperidin | 1,05 | Destillation | 1-[2'-(1",3"-Dioxobutyloxy)-propyl]-2,2,6,6-tetramethyl-piperidin | kp 136-137°/0,8 mmHg |
| 12 | 2,2,6,6-Tetramethyl-4-(N-äthylamino)-piperidin | 1,05 | Destillation | 2,2,6,6-Tetramethyl-4-[N-äthylamino-N-(1',3'-dioxobutyl)-piperidin | kp 117-118°/0,1 mmHg |
| 13 | 1-Allyl-2,2,6,6-tetramethyl-4-hydroxy-piperidin | 1,05 | Destillation | 1-Allyl-2,2,6,6-tetramethyl-4-(1',3'-dioxobutyloxy)-piperidin | kp 136-137°/0,8 mmHg<br>gef. C 68,5% H 9,8% N 5,0%<br>ber. C 68,29% H 9,67% N 4,98% |
| 14 | 1-n-Butyl-2,2,6,6-tetramethyl-4-hydroxy-piperidin | 1,05 | Destillation | 1-n-Butyl-2,2,6,6-tetramethyl-4-(1',3'-dioxobutyloxy)-piperidin | kp 147-148°/1,0 mmHg<br>gef. C 68,9% H 10,5% N 4,8%<br>ber. C 68,88% H 10,20% N 4,73% |
| 15 | 1,4-Bis(2',2'6',6'-tetramethyl-4'-hydroxy-piperidino)-buten(2) | 2,10 | Umkristallisation aus Aether | 1,4-Bis [2',2',6',6'-tetramethyl-4'-(1",3"-dioxobutyloxy)-piperidino]-buten(2) | F 126-128°<br>gef. C 67,4% H 9,3% N 5,5%<br>ber. C 67,38% H 9,43% N 5,24% |
| 16 | Bis-[1-(2'-hydroxyäthyl)-2,2,6,6-tetramethyl-piperidin-4-yl]-terephthalat | 2,10 | Umkristallisation aus Acetonitril | Bis [1-(3'-oxa-4',6'-dioxo-heptyl)-2,2,6,6-tetramethyl-piperidin-4-yl]-terephthalat | F 124-125° |

## Beispiele 17 - 26

Die Herstellung der Verbindungen der Beispiele 17-26 (siehe Tabelle 2) erfolgte nach der folgenden allgemeinen Vorschrift.

0,1 Mole der in Kolonne 2 bezeichneten Verbindungen wurden in 200 ml Methanol gelöst und mit 100 ml einer 1,0 molaren methanolischen Natriummethylatlösung versetzt. Zu diesen Lösungen wurden 0,05 Mole der in Kolonne 3 bezeichneten Metallsalze, gelöst in 160 ml Methanol, innerhalb von 30 Minuten zugetropft. Das Reaktionsgemisch wurde darauf 30 Minuten unter Rückfluss erhitzt und das ausgefallene Neutralsalz durch Filtration abgetrennt. Im Beispiel 14 wurde das Reaktionsgemisch nach Zugabe des Metallsalzes nicht erhitzt, wobei sich kein Neutralsalz abschied. Die erhaltenen Lösungen wurden zur Trockne eingedampft und der Rückstand mit dem in Kolonne 4 bezeichneten Extraktionsmittel eluiert. Der Extrakt wurde eingedampft und bei einer Temperatur von 60° (Ausnahme Beispiel 14: 40°) und einem Druck von 11 mm Hg 10 Stunden getrocknet. Man erhielt auf diese Weise die in Kolonne 5 bezeichneten festen Endprodukte, deren Eigenschaften in Kolonne 6 genannt werden.

Tabelle 2

| 1. Bei-spiel | 2. Ausgangsprodukte Verbindungen nach Beispiel | 3. Metallsalze | 4. Extraktionsmittel |
|---|---|---|---|
| 17 | 1 | $NiCl_2$ | Methylenchlorid |
| 18 | 3 | $NiCl_2$ | Chloroform |
| 19 | 4 | $NiCl_2$ | Methylenchlorid |
| 20 | 4 | $Mg(CH_3COO^-)_2 \cdot 4\,H_2O$ | Methylenchlorid |
| 21 | 5 | $ZnCl_2$ | Hexan |
| 22 | 6 | $NiCl_2$ | Hexan |
| 23 | 8 | $NiCl_2$ | Aether |

Fortsetzung
Tabelle 2

| Beispiel | 5. Endprodukte | 6. Eigenschaften Farbe | |
|---|---|---|---|
| 17 | 1:2 Ni-Chelat des 1-[2' 1",3"-Dioxobutyloxy)-äthyl]-2,2,6,6-tetramethylpiperidin-Enolates | hellgrün | Ni Gehalt 9,4%<br>N Gehalt 3,75% |
| 18 | 1:2 Ni-Chelat des 2,2,6,6-Tetramethyl-4-(1',3'-dioxobutyloxy)-piperidin-Enolates | hellgrün | Ni Gehalt 10,1%<br>N Gehalt 5,1% |
| 19 | 1:2 Ni-Chelat des 1,2,2,6,6-Pentamethyl-4-(1',3'-dioxobutyloxy)-piperidin-Enolates | hellgrün | Ni Gehalt 9,9%<br>N Gehalt 4,95% |
| 20 | 1:2 Mg (II)-Chelat des 1,2,2,6,6-Pentamethyl-4-(1',3'-dioxobutyloxy)-piperidin-Enolates mit 13/4 Mol $H_2O$ pro $Mg^{2+}$ | weiss | F. 221-225°<br>Elementargehalt in %<br>gef. C 59,62% H 9,08% Mg 4,03%<br>ber. 59,57% 9,19% 4,30% |
| 21 | 1:2 Zn II-Chelat des 2,2,6,6-Tetramethyl-4-[N-n-butylamin-N-(1',3'-dioxobutyl]-piperidin-Enolates | weiss | Zn Gehalt 10,0%<br>N Gehalt 8,5% |
| 22 | 1:2 Ni-Chelat des 1,2,2,6,6-Pentamethyl-4-[N-n-heptyl-amino-N-(1',3'-dioxobutyl)]-piperidin-Enolates | hellgrün | Ni Gehalt 7,5%<br>N Gehalt 7,6% |
| 23 | 1:2 Ni-Chelat des 2-(1',3'-Dioxobutyloxymethyl)-7,7,9,9-tetramethyl-1,4-dioxy-8-aza-spiro[4.5]-decan-Enolates | hellgrün | Ni Gehalt 8,1%<br>N Gehalt 4,25% |

Fortsetzung Tabelle 2

| 1. Beispiel | 2. Ausgangsprodukte Verbindungen nach Beispiel | 3. Metallsalze | 4. Extraktionsmittel | 5. Endprodukte | 6. Eigenschaften/Farbe |
|---|---|---|---|---|---|
| 24 | 10 | $NiCl_2$ | Aether | 1:2-Ni-Chelat des 1-[2'-(1",3"-Dioxobutyloxy)propyl]-2,2,6,6-tetramethylpiperidin-Enolates | hellgrün<br>Ni Gehalt 9,8 %<br>N Gehalt 4,4 % |
| 25 | 11 | $NiCl_2$ | Hexan | 1:2 Ni-Chelat des 2,2,6,6-Tetramethyl-4-[N-äthylamino-N-(1',3'-dioxobutyl)]-piperidin-Enolates | hellgrün<br>Ni Gehalt 8,5 %<br>N Gehalt 9,1 % |
| 26 | 12 | $NiCl_2$ | Hexan | 1:2 Ni-Chelat des 1-Allyl-2,2,6,6-tetramethyl-4-(1',3'-dioxobutyloxy)-piperidin-Enolates | hellgrün<br>Ni Gehalt 8,7 %<br>N Gehalt 4,5 % |

## Beispiel 27

18,5 g (0,05 Mol) 1-[2'-(1",3"-Dioxobutyloxy)-äthyl]-4-(1",3"-dioxobutyloxy)-2,2,6,6-tetramethylpiperidin wurden in 200 ml Methanol gelöst und 5,40 g (0,1 Mol) Natriummethylat hinzgefügt.

In diese Lösung tropfte man im Verlaufe von 30 Minuten 16,3 g (0,05 Mol) in 330 ml Benzol gelöstes Nickel(II)-Oenanthat (das pro Mol 1/2 Mol Wasser enthält) ein. Das Gemisch wurde 4 Stunden bei Raumtemperatur gerührt, durch Filtration geklärt und zur Trockne eingedampft. Der Rückstand wurde mit Wasser gewaschen und bei einer Temperatur von 60° und einem Druck von 11 mm Hg 18 Stunden getrocknet. Auf diese Weise erhielt man den 1:1 Nickel(II)-Komplex des 1-[2'-(1",3"-Dioxobutyloxy)-äthyl]-4-(1",3"-dioxobutyloxy)-2,2,6,6-tetramethylpiperidin-Dienolats als ein Monohydrat in Form eines gelbgrünen Pulvers.

Elementaranalyse:

| | | | | |
|---|---|---|---|---|
| gef. | C 51,40% | H 7,22% | N 2,92% | Ni 13,0% |
| ber. für Monohydrat | 51,37% | 7,02% | 3,15% | 13,2%. |

## Beispiel 28

25,3 g (0,05 Mol) N,N'-Bis-(1',3'-dioxobutyl)-N,N'-bis-(2,2,6,6-tetramethyl-4-piperidyl)-äthylendiamin wurden in 500 ml Toluol gelöst und mit 76,9 ml einer 1,3 molaren Natriummethylatlösung (0,1 Mol) versetzt. Unter Rückfluss wurden nun 16,3 g (0,05 Mol)in 330 ml Toluol gelöstes Nickel(II)-Oenanthat (das pro Mol 1/2 Mol Wasser enthält) zugetropft und das Reaktionsgemisch 15 Minuten unter Rückfluss gekocht. Das sich hierbei abscheidende Natriumoenanthat wurde abfiltriert und das Filtrat zur Trockne eingedampft. Der Rückstand wurde bei Raumtemperatur mit Toluol extrahiert, im Vakuum eingedampft und bei einer Temperatur von 60° und einem Druck von 11 mm Hg 10 Stunden getrocknet. Man erhielt auf diese Weise den 1:1 Nickel-Komplex des N,N'-Bis-(1',3'-dioxobutyl)-N,N'-bis-(2,2,6,6-tetramethyl-4-piperidyl)-äthylendiamin-Dienols als hellgrünes Pulver, das 9,4% Nickel und 9,2% Stickstoff enthält und in das neben dem genannten Liganden Oenanthsäureanionen koordinativ eingebaut sind.

## Beispiel 29

12,1 g (0,05 Mol) 2,2,6,6-Tetramethyl-4-(1',3'-dioxobutyloxy)-piperidin wurden in 400 ml Aethanol gelöst und 3,40 g (0,0166 Mol) Aluminiumisopropylat zugefügt. Bei Erwärmen der Mischung erhält man eine klare Lösung, die zwei Stunden unter Rückfluss erhitzt wird. Dann wird das Lösungsmittel und das freigewordene Isopropanol abgedampft und das Produkt bei einer Temperatur von 60° C und einem Druck von 11 mmHg getrocknet. Auf diese Weise erhält man den 1:3 Aluminium (II)-Komplex des 2,2,6,6-Tetramethyl-4-(1',3'-dioxobutyloxy)-piperidin-Enolates als ein weisses Pulver.

Elementaranalyse

| | | | | |
|---|---|---|---|---|
| gef. | C 62,33 % | H 9,03 % | N 5,64 % | Al 3,73 % |
| ber. | C 62,63 % | H 8,89 % | N 5,62 % | Al 3,61 % |

## Beispiel 30

Lichtschutzwirkung in PP-Fasern

1000 Teile unstabilisiertes Polypropylenpulver (Schmelzindex ~18) werden in einem Trommelmischer mit 1 Teil Pentaerithrytyl-tetrakis [3-(3.5-di-tert.-butyl-4-hydroxylphenyl)-propionat] und mit 3 Teilen der in der Tabelle angeführten Additive gemischt und anschliessend in einem Extruder bei 220° C extrudiert und granuliert. Das erhaltene Granulat wird in einer Labor-Schmelzspinnanlage bei einer maximalen Temperatur von 270° C und einer Geschwindigkeit von 300 m/Minute zu einem 130/37 denier Multifilament versponnen. Dieses wird verstreckt und gezwirnt mittels einer Streckzwirnmaschine. Das Streckverhältnis ist 1:5,6 und die Zwirnzahl 15/Meter, so dass schliesslich Multifilamente 130/37 denier erhalten werden. Diese Multifilamente werden auf weissen Karton montiert und im Xenotest 1200 belichtet.

Als Mass der Schutzwirkung gilt die Belichtungszeit bis 50 % Reissfestigkeitsverlust.

Die Ergebnisse sind in der Tabelle 3 zusammengefasst.

Tabelle 3

| Additiv | Zeit bis die Reissfestigkeit durch Belichtung im Xenotest 1200 auf die Hälfte des Ausgangswertes reduziert |
|---|---|
| keines | 350 Stunden |
| Verbindung 19 | 2050 Stunden |

## Beispiel 31

### Lichtschutzwirkung in Hochdruckpolyäthylen-Folien

100 Teile Polyäthylengranulat niederer Dichte (= 0.917) werden mit 0.05 Teilen eines Stabilisators der folgenden Tabelle im Brabenderplastographen bei 180° C während 10 Minuten homogenisiert. Die so erhaltene Masse wird möglichst rasch dem Kneter entnommen und in einer Kniehebelpresse zu einer 2-3 mm dicken Platte gepresst. Ein Teil des erhaltenen Rohpresslings wird ausgeschnitten und zwischen zwei Hochglanz-Hartaluminiumfolien mit einer handhydraulischen Laborpresse während 6 Minuten bei 170° und 12 Tonnen Druck zu einer 0.1 mm dicken Folie gepresst, die unverzüglich in kaltem Wasser abgeschreckt wird. Aus dieser werden nun Abschnitte von je 60 x 44 mm gestanzt und im Xenotest 1200 belichtet. Zu regelmässigen Zeitabständen werden diese Prüflinge aus dem Belichtungsapparat entnommen und in einem IR-Spektrophotometer auf ihren Carbonylgehalt geprüft. Die Zunahme der Carbonylextinktion bei der Belichtung ist ein Mass für den photooxidativen Abbau des Polymeren (s. L. Blaban et al., J. Polymer Sci. Part C, 22, 1059 - 1071 (1969); J.F. Heacock, J. Polymer Sci. Part. A-1, 22, 2921-34 (1969); D.J. Carlsson and DM. Wiles, Macromolecules 2, 587-606 (1969) und ist erfahrungsgemäss mit einem Abfall der mechanischen Eigenschaften des Polymeren verbunden.

Als Mass der Schutzwirkung gilt die Zeit bis Erreichen einer Carbonylextinktion von 0.100.

Die Ergebnisse sind in der Tabelle 4 zusammengefasst.

BAD ORIGINAL

Tabelle 4

| Additiv | Belichtungszeit im Xenotest 1200 bis eine Carbonylextinktion von 0,1 nachgewiesen wurde |
|---|---|
| keines | 270 Stunden |
| Verbindung 17 | 800 Stunden |
| 20 | 1840 Stunden |
| 22 | 2300 Stunden |

## Beispiel 32

Lichtschutz von Polyäthylen-Blasfolien

100 Teile Polyäthylen niedriger Dichte (Schmelzindex 0,1-0,3 g/10 Min. , 190° C, 2,16 kg) wurden 2 Minuten in einem gekühlten Intensivmischer der Firma Henschel mit einem der in der nachstehenden Tabelle 1 aufgeführten Zusätze und 0,03 Teilen 3(3',5'Ditertiärbutyl 4'hydroxyphenyl)octadecylpropionat gemischt, aus einem Einschneckenextruder mit 35 mm Durchmesser und 700 mm Länge extrudiert, wobei die Temperatur im ersten Abschnitt auf 190° C und in den folgenden drei auf 200° C gestellt wurde, in Wasser gekühlt und granuliert. Dieses Granulat wurde bei 190° C durch einen Blasfolienextruder von 60 mm Durchmesser und 1200 mm Länge gepresst. Am Ausgang des Extruders war eine Ringdüse mit 120 mm Druchmesser angeordnet, die auf 200° C eingestellt wurde. Die schlauchartig ausgestossene Folie wurde im Verhältnis von 1:1,3 aufgeblasen. Die Abzuggeschwindigkeit betrug 5 m/Minute. Die Folien waren 0,2 mm dick.

Diese Folien wurden im Xenotest-Belichtungsgerät 1200 belichtet.

In regelmässigen Abständen wurde in einem Infrarot-Spektrophotometer die Carbonylextinktion der Folien gemessen.

Die durch die Carbonylextinktion angezeigte Oxydation entspricht im Bereich von 0,1 einer mechanischen Schädigung der Folie, welche den Beginn der Unbrauchbarkeit anzeigt.

Als Mass der Stabilisierung wird in der nachfolgenden Tabelle die Belichtungszeit angegeben, welche die Folie erträgt, bis sie die erwähnte Carbonylextinktion von 0,1 erreicht.

Tabelle 5

| Additiv | Belichtungszeit im Xenotest 1200 bis eine Carbonylextinktion von 0,1 nachgewiesen wurde |
|---|---|
| keines | 320 Stunden |
| Verbindung 19 | 3650 Stunden |

## Beispiel 33

### Stabilisierung von Polypropylen gegen Licht

100 Teile Polypropylenpulver (Moplen, Fibre grade, der Firma Montedison) werden mit 0,2 Teilen β-(3,5-Di-tert.-butyl-4-hydroxyphenyl)-propionsäure-octadecylester und 0,25 Teilen eines Stabilisators der folgenden Tabelle 1 im Brabender-Plastographen bei 200° C während 10 Minuten homogenisiert. Die so erhaltene Masse wird möglichst rasch dem Kneter entnommen und in einer Kniehebelpresse zu einer 2-3 mm dicken Platte gepresst. Ein Teil des erhaltenen Presslings wird ausgeschnitten und zwischen zwei Hochglanz-Hartaluminiumfolien mit einer handhydraulischen Laborpresse während 6 Minuten bei 260° C und 12 Tonnen Druck zu einer 0,1 mm dicken Folie gepresst, die unverzüglich in kaltem Wasser abgeschreckt wird. Aus dieser werden nun Abschnitte von je 60 x 44 mm gestanzt und im Xenotest 150 belichtet. Zu regelmässigen Zeitabständen werden diese Prüflinge aus dem Belichtungsapparat entnommen und in einem IR-Spektrophotometer auf ihren Carbonylgehalt geprüft. Die Zunahme der Carbonylextinktion bei 5,85 µ während der Belichtung ist ein Mass für den photoxidativen Abbau des Polymeren (s. L. Blaban et al., J. Polymer Sci, Part C; 22, 1059-1071 (1969) und ist erfahrungsgemäss mit einem Abfall der mechanischen Eigenschften des Polymeren verbunden. Als Mass der Schutzwirkung gilt die Zeit bis Erreichen einer Carbonylextinktion von ca. 0,3 , bei welcher die Vergleichsfolie brüchig ist.

Die Schutzwirkung der Stabilisatoren gemäss Erfindung ist aus folgender Tabelle 1 ersichtlich:

| Additiv | Belichtungszeit im Xenotest 150 bis eine Carbonylextinktion von 0,3 nachgewiesen wurde |
|---|---|
| keines | 1050 |
| Verbindung 1 | 3300 |
| Verbindung 4 | 3900 |

Polypropylen Bändchen

100 Teile Polypropylenpulver (Schmelzindex 1,5 g/10 Minuten, 230° C, 2160 g) wurden in einem Trommelmischer mit 0,1 Teil Pentacrythrit-tetrakis[3-(3',5'-ditertiärbutyl-4-hydroxyphenyl)-propionat] und 0,15 Teile der in der nachfolgenden Tabelle angeführten Additive gemischt und in einem Extruder bei 200 bis 220° C extrudiert und granuliert. Das erhaltene Granulat wurde in üblicher Weise mittels eines Extruders mit Breitschlitzdüse zu Folien verarbeitet, die in Bändchen geschnitten wurden, welche anschliessend bei erhöhter Temperatur auf die sechsfache Länge verstreckt wurden. Der Titer dieser Bändchen beträgt 700 - 900 den, ihre Breite 4 mm ihre Reissfestigkeit 5,5-6,5 g/den. Diese Bändchen wurden in einem Xenotest 1200 belichtet. In regelmässigen Abständen wurden Proben einem Zug-Dehnungsversuch unterworfen, wobei sich mit zunehmender Belichtung eine fortschreitende Abnahme der Reissfestigkeit ergibt. Die Belichtungszeit bis zum Abfall der Reissfestigkeit auf die Hälfte des Ausgangswertes wird durch die Wirkung der Lichtschutzmittel verlängert.

| Additiv | Belichtungszeit im Xenotext 1200 bis die Reissfestigkeit auf die Hälfte des Ausgangswertes reduziert wurde |
|---|---|
| keines | 400 Stunden |
| Verbindung 9 | 4000 Stunden |
| 17 | 2800 Stunden |
| 18 | >5000 Stunden |
| 19 | 3250 Stunden |
| 21 | 825 Stunden |
| 22 | 3000 Stunden |
| 28 | 2400 Stunden |
| 29 | >3500 Stunden |

## Patentansprüche

1. Neue Verbindungen der allgemeinen Formel I

$$M^{q\oplus} \left[L^{r\ominus}\right]_{q/r} \cdot m\,A \qquad (I),$$

worin

| | |
|---|---|
| $M^{q\oplus}$ | ein zweifach oder dreifach positiv geladenes Metallion ist, und |
| q | 2 oder 3 ist, und |
| r | 1 oder 2 ist, wobei der Quotient q/r 1, 1.5, 2 oder 3 ist, und |
| L | ein durch mindestens eine Enolatgruppe in 1- oder 4-Stellung substituiertes Piperidin dessen Ringstickstoff durch Alkylsubstituenten an den benachbarten Stellungen sterisch gehindert ist bedeuten, und |
| m | 0, 1 oder 2 ist, und |
| A | $H_2O$ oder ein Amin ist. |

2. Neue Verbindungen gemäss Anspruch 1 der Formel I worin

| | |
|---|---|
| $M^{q\oplus}$ | ein zweifach oder dreifach positiv geladenes Metallion ist, und |

q 2 oder 3 ist, und

r 1 oder 2 ist, wobei der Quotient q/r 1, 1.5, 2 oder 3 bedeutet, und

L eine Gruppe der Formel II

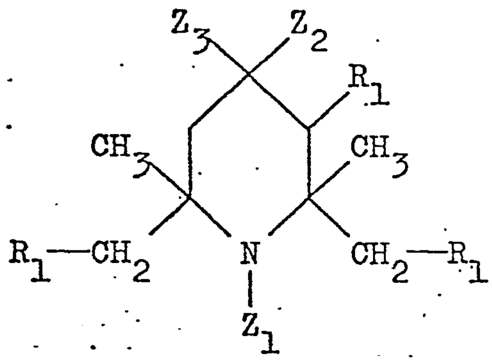

(II)

ist, worin

$R_1$ Wasserstoff oder $C_1$-$C_4$ Alkyl ist, und

$Z_1$ Wasserstoff, $C_1$-$C_{12}$ Alkyl, $C_3$-$C_8$ Alkenyl, $C_3$-$C_4$ Alkinyl, $C_2$-$C_{21}$ Alkoxyalkyl, $C_7$-$C_8$ Aralkyl, eine aliphatische Acylgruppe mit 1-4 C-Atomen, oder eine der Gruppen $-CH_2COOR_2$ oder $-CH_2-CH(R_3)-OR_4$, oder einen Rest $-(CH_2)_4-R_5$, $-CH_2-CH=CH-CH_2-R_5$, $-CH_2-\langle O \rangle-CH_2-R_5$, $-CH_2-\langle O \rangle-\langle O \rangle-CH_2-R_5$, oder eine Gruppe der Formel III

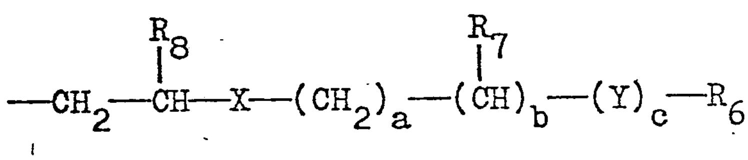

(III)

bedeutet, wobei

$R_2$ $C_1$-$C_8$ Alkyl, $C_3$-$C_6$ Alkenyl, Phenyl, $C_7$-$C_8$ Aralkyl oder Cyclohexyl ist, und

$R_3$ Wasserstoff, Methyl oder Phenyl ist, und

$R_4$ eine aliphatische $C_1$-$C_{18}$ Acylgruppe, eine aromatische $C_7$ Acylgruppe, eine araliphatische $C_8$-$C_9$ Acylgruppe oder eine alicyclische $C_6$-$C_9$ Acylgruppe bedeutet, wobei der aromatische Teil gegebenenfalls mit Chlor, $C_1$-$C_4$ Alkyl, $C_1$-$C_8$ Alkoxy, und/oder Hydroxy substituiert sein kann,bedeutet,

und

$R_5$ eine Gruppe der Formel IV

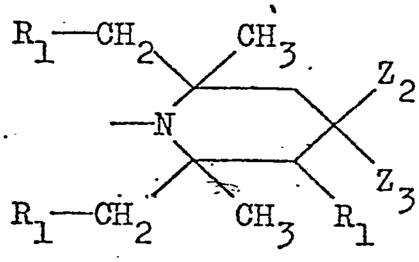

(IV)

bedeutet, worin $R_1$, $Z_2$ und $Z_3$ die hier angegebene Bedeutung haben, und

$R_6$ eine Gruppe der Formel V

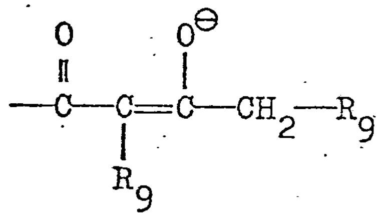

(V)

ist, worin

$R_9$ Wasserstoff oder $C_1$-$C_4$ Alkyl ist, und

$R_7$ und $R_8$ unabhängig voneinander Wasserstoff, Methyl, Phenyl, $C_2$-$C_9$ Alkoxymethyl oder ein Rest $-CH_2-O-R_6$ ist, worin $R_6$ oben definierte Bedeutung hat, und

a 0, 1 oder 2, und

b 0 oder 1 ist, und

c 0 oder, falls a und/oder b von 0 verschieden sind, auch 1 ist, und

X und Y unabhängig voneinander -O- oder $-\overset{|}{N}R_{10}$, worin

$R_{10}$ Wasserstoff, $C_1$-$C_{12}$ Alkyl, $C_3$-$C_8$ Alkenyl, Cyclo-

hexyl, $C_3$-$C_{21}$ Alkoxyalkyl, $C_7$-$C_{13}$ Aralkyl, gegebenenfalls durch $C_1$-$C_8$ Alkyl und/oder $C_1$-$C_8$ Alkoxy substituiertes Phenyl oder eine Gruppe $-CH_2-CH_2-OR_6$, worin $R_6$ die oben angegebene Bedeutung hat, ist, und $R_{10}$ ferner eine Gruppe der Formel IV

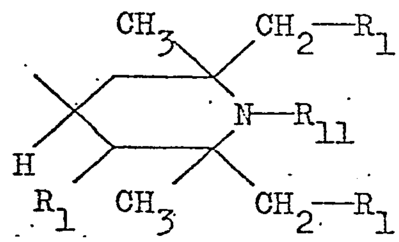

(VI) ,

worin

$R_1$ die oben angegebene Bedeutung hat, und

$R_{11}$ Wasserstoff, $C_1$-$C_{12}$ Alkyl, $C_3$-$C_8$ Alkenyl, $C_2$-$C_{21}$ Alkoxyalkyl, $C_7$-$C_8$ Aralkyl, eine aliphatische Acylgruppe mit 1-4 C-Atomen oder eine Gruppe $-CH_2COOR_2$, worin $R_2$ die oben definierte Bedeutung hat, bedeutet, und

$Z_2$ Wasserstoff, $C_2$-$C_{18}$ aliphatisches Acyloxy, gegebenenfalls durch $C_1$-$C_8$ Alkyl oder $C_1$-$C_8$ Alkoxy substituiertes Benzoyloxy oder eine gegebenenfalls $C_1$-$C_8$ N-alkyl-substituierte $C_2$-$C_{18}$ Acylamidogruppe ist, oder eine der Gruppen $-R_{12}$, $-(CH_2)_d-R_{12}$, $-CH_2$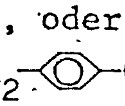$-CH_2-R_{12}$ oder $-CH_2$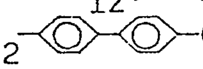$-CH_2-R_{12}$ ist, worin d 2 bis 10 ist und $R_{12}$ eine Gruppe der Formel VII

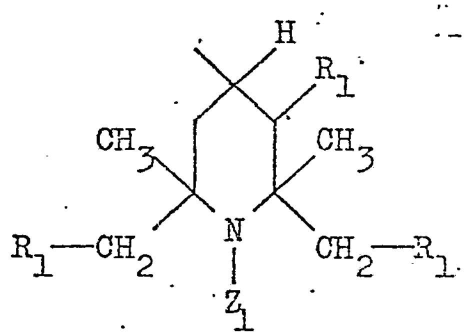

(VII)

ist, worin

$R_1$ und $Z_1$ die oben angegebene Bedeutung haben, oder

$Z_2$ ferner eine Gruppe der Formel VIII

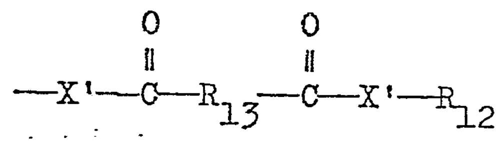

(VIII) ,

darstellt, worin

$R_{12}$ die oben angegebene Bedeutung hat, und

X' -O- oder $-\overset{|}{N}-R_{14}$ ist, wobei

$R_{14}$ Wasserstoff oder $C_1$-$C_4$ Alkyl bedeutet, und

$R_{13}$ $-(CH_2)_e-$ oder eine Gruppe

$R_{15}$
-C- ist, wobei e 0 bis 8 ist, und
$R_{16}$

$R_{15}$ und $R_{16}$ unabhängig voneinander Wasserstoff, $C_1$-$C_{12}$ Alkyl, $C_3$-$C_8$ Alkenyl, $C_7$-$C_{11}$ Aralkyl, Cyanomethyl, Cyanoäthyl oder eine Gruppe $-CH_2COOR_{17}$, wobei

$R_{17}$ Methyl oder Aethyl ist, bedeuten, oder

$Z_2$ eine der Gruppen

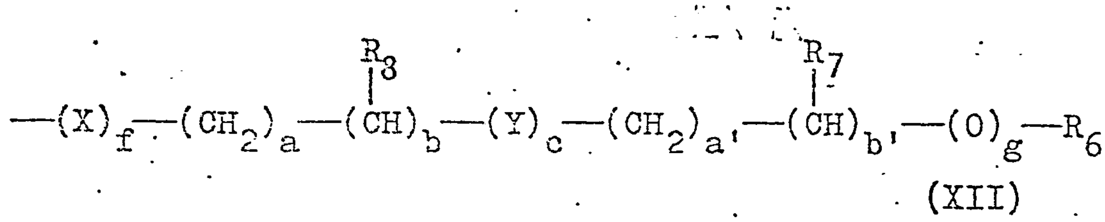

(XII)

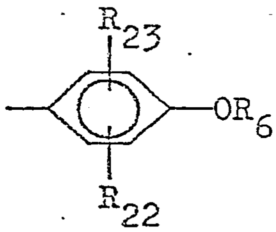 (XIII) oder 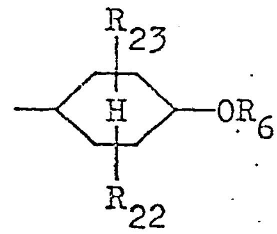 (XIV)

ist, worin a, b, c, X, Y, $R_6$, $R_7$ und $R_8$ die oben angegebene Bedeutung haben, und

f,a'undb' 0 oder 1 sind, und

g 0 oder falls a' und/oder b' von 0 verschieden sind, auch 1 ist, und

$R_{22}$und$R_{23}$ unabhängig voneinander Wasserstoff, $C_1$-$C_4$ Alkyl, Cyclohexyl, Phenyl, Benzyl, α-Methylbenzyl oder α,α-Dimethylbenzyl bedeuten, und

$Z_3$ Wasserstoff oder Cyano ist, oder

$Z_2$ und $Z_3$ zusammen eine der Gruppen XV, XVI oder XVII

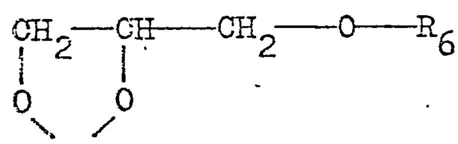

(XV)

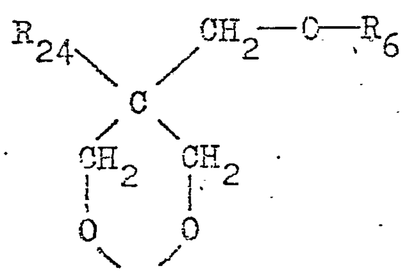

(XVI) oder

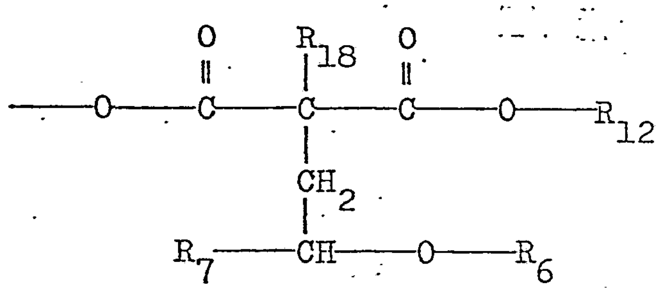

(IX)

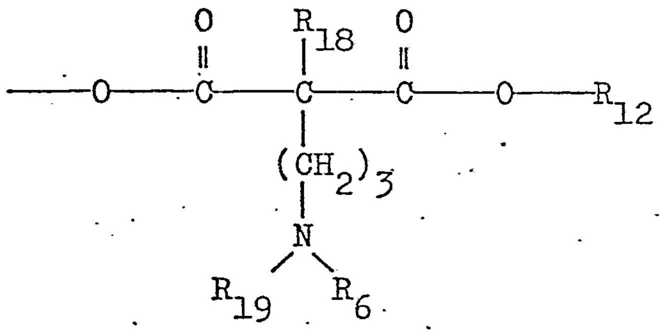

(X) oder

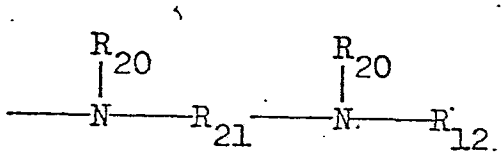

(XI) bedeutet

worin

$R_6$, $R_7$ und $R_{12}$ die oben angegebene Bedeutung haben und

$R_{18}$ Wasserstoff, $C_1$-$C_8$ Alkyl, Allyl, Phenyl oder Benzyl ist, und

$R_{19}$ Wasserstoff, $C_1$-$C_8$ Alkyl, Allyl oder Benzyl ist, und

$R_{20}$ $C_1$-$C_4$ Alkyl oder eine Gruppe der Formel V mit oben angegebener Bedeutung ist und

$R_{21}$ $C_2$-$C_6$ Alkylen, Cyclohexyliden,Phenylen,Diphenylen, 4,4'-Diphenylenoxyd oder 4,4'-Diphenylenmethan ist, oder

$Z_2$ ferner eine Gruppe der Formeln XII, XIII oder XIV

$R_{29}$ Wasserstoff oder Alkyl bedeutet.

3. Verbindungen gemäss Anspruch 1 der Formel I, worin

$M^{q\oplus}$ $Mg^{2+}$, $Ca^{2+}$, $Sr^{2+}$, $Ba^{2+}$, $Zn^{2+}$, $Cd^{2+}$, $Al^{3+}$, $Sn^{2+}$, $Cr^{3+}$, $Co^{2+}$, $Ni^{2+}$, $VO^{2+}$, $MoO^{2+}$, $(R^*)_2Sn^{2+}$ oder $(CH_2CH_2COOR^*)_2Sn^{2+}$ bedeutet, worin

$R^*$ $C_1$-$C_8$ Alkyl ist, und

q 2 oder 3 ist, und

r 1 oder 2 ist, wobei der Quotient q/r 1, 1.5, 2 oder 3 bedeutet, und

L eine Gruppe der Formel II ist, worin

$R_1$ Wasserstoff oder Methyl ist, und

$Z_1$ Wasserstoff, $C_1$-$C_8$ Alkyl, $C_3$-$C_4$ Alkenyl, $C_3$ Alkinyl, $C_2$-$C_{10}$ Alkoxyalkyl, $C_7$-$C_8$ Aralkyl, Acetyl oder eine der Gruppen $-CH_2COOR_2$ oder $-CH_2-CH(R_3)-OR_4$, oder einen Rest $-(CH_2)_4-R_5$, $-CH_2-CH=CH-CH_2-R_5$, $-CH_2-$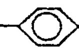$-CH_2-R_5$, $-CH_2-$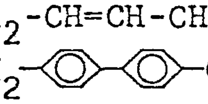$-CH_2-R_5$, oder eine Gruppe der Formel III bedeutet, wobei

$R_2$ $C_1$-$C_4$ Alkyl, $C_3$-$C_4$ Alkenyl, Phenyl, $C_7$-$C_8$ Aralkyl oder Cyclohexyl ist, und

$R_3$ Wasserstoff oder Methyl ist,

$R_4$ eine aliphatische Acylgruppe mit 1-12 C-Atomen ist, und

$R_5$ eine Gruppe der Formel IV ist, worin $Z_2$ und $Z_3$ die unten angegebene Bedeutung haben und $R_1$ Wasserstoff ist, und

$R_6$ eine Gruppe der Formel V bedeutet, worin $R_9$ Wasserstoff oder Methyl ist, und

$R_7$ und $R_8$ unabhängig voneinander Wasserstoff oder Methyl sind, und

a 0, 1 oder 2, und

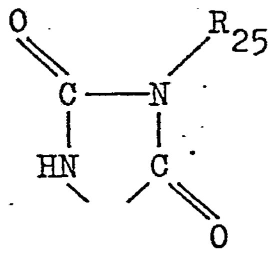

(XVII) bedeuten, worin

| | |
|---|---|
| $R_6$ | oben angegebene Bedeutung hat, und |
| $R_{24}$ | Wasserstoff, Methyl oder Aethyl ist, und |
| $R_{25}$ | Wasserstoff, $C_1$-$C_{18}$ Alkyl, $C_3$-$C_8$ Alkenyl, Cyclohexyl, $C_7$-$C_8$ Aralkyl oder eine Gruppe $-CH_2-COOR_{26}$ oder $-CH_2-CH(R_8)-O-R_6$, bedeutet, wobei |
| $R_{26}$ | $C_1$-$C_{18}$ Alkyl ist, und $R_6$ und $R_8$ die oben angegebene Bedeutung haben, und |
| m | 0, 1 oder 2 ist, und |
| A | $H_2O$ oder ein Amin der Formel (XVIII) |

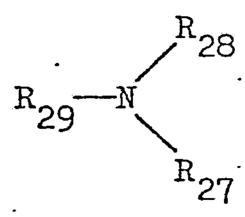

(XVIII)

ist, worin

| | |
|---|---|
| $R_{27}$ | gegebenenfalls substituiertes Alkyl, Cycloalkyl, Aryl, Aralkyl, eine gegebenenfalls substituierte Aminoalkyl- oder Piperidinylgruppe ist, und |
| $R_{28}$ | Wasserstoff oder gegebenenfalls substituiertes Alkyl, Cycloalkyl, eine gegebenenfalls substituierte Aminoalkylgruppe oder eine Piperidinylgruppe bedeutet, oder |
| $R_{27}$ und $R_{28}$ | zusammen mit dem N-Atom einen durch Alkylgruppen substituierten oder unsubstituierten Pyrrolidin-, Piperidin- oder Morpholinring bilden, und |

worin $R_6$ und $R_7$ die oben angegebene Bedeutung haben, und

$R_{18}$ Wasserstoff bedeutet,

$R_{19}$ Wasserstoff, $C_1$-$C_4$ Alkyl, Allyl oder Benzyl ist,

$R_{20}$ $C_1$-$C_4$ Alkyl oder eine Gruppe der Formel V mit oben angegebener Bedeutung ist, und

$R_{21}$ $C_2$-$C_6$ Alkylen, Cyclohexyliden oder Phenylen ist oder

$Z_2$ ferner eine Gruppe der Formel XIIa

$$(X_1)_f-(CH_2)_a-(\overset{R_7}{\overset{|}{C}}H)_b-(Y_1)_c-R_6 \qquad (XIIa)$$

ist, worin

a, b, c, $R_6$ und $R_7$ oben angegebene Bedeutung haben, und

f 0 oder 1 ist, und

$X_1$ -O- oder $-\overset{|}{N}-R^*_{10}$ bedeutet, worin

$R^*_{10}$ Wasserstoff, $C_1$-$C_{12}$ Alkyl, Cyclohexyl, $C_3$-$C_{14}$ Alkoxyalkyl, $C_7$-$C_8$ Aralkyl, gegebenenfalls mit $C_1$-$C_3$ Alkyl und/oder $C_1$-$C_8$ Alkoxy mono- oder disubstituiertes Phenyl oder falls a, b und c 1, $R_7$ Wasserstoff und Y -O- sind, auch eine Gruppe $-CH_2-CH_2-OR_6$, worin $R_6$ die oben angegebene Bedeutung hat, ist, und $R^*_{10}$ ferner eine Gruppe VI bedeutet, worin

$R_1$ die oben angegebene Bedeutung hat, und

$R_{11}$ Wasserstoff, Methyl oder Allyl ist, und

$Y_1$ -O- oder $-\overset{|}{N}-R^{**}_{10}$ bedeutet, worin

$R^{**}_{10}$ Wasserstoff, $C_1$-$C_8$ Alkyl oder Phenyl ist, oder

$Z_2$ ferner eine der Gruppen XIII oder XIV ist , worin

$R_6$ die oben angegebene Bedeutung hat, und

$R_{22}$ und $R_{23}$ unabhängig voneinander Wasserstoff, Methyl oder Aethyl sind, und

$Z_3$ Wasserstoff oder Cyano ist, oder

b 0 oder 1, und

c 0 und falls a und/oder b verschieden von 0 sind, auch 1 ist, und

X -O- ist, und

Y -O- oder $-\overset{|}{N}-R_{10}$ ist, wobei

$R_{10}$ Wasserstoff oder Methyl, oder falls a, b und c 1, $R_7$ Wasserstoff und Y -O- sind, auch eine Gruppe $-CH_2-CH_2-OR_6$, worin $R_6$ die oben angegebene Bedeutung hat, bedeutet, und

$Z_2$ Wasserstoff, $C_2$-$C_{12}$ aliphatisches Acyloxy, gegebenenfalls mit Methyl oder Methoxy mono- oder disubstituiertes Benzoyloxy, oder eine gegebenenfalls $C_1$-$C_4$ N-alkylsubstituierte $C_2$-$C_{12}$ Acylamidogruppe ist, oder eine der Gruppen $-R_{12}$, $-(CH_2)_d-R_{12}$, 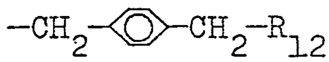 oder 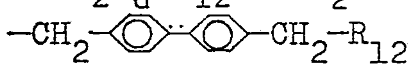 ist, worin d 2 bis 6 ist, und

$R_{12}$ eine Gruppe der Formel VII ist, worin $R_1$ und $Z_1$ oben angegebene Bedeutung haben, oder

$Z_2$ ferner eine Gruppe der Formel VIII bedeutet, worin

X' -O- oder $-\overset{|}{N}-R_{14}$ ist, wobei

$R_{14}$ Wasserstoff oder $C_1$-$C_4$ Alkyl ist, und

$R_{13}$ $-(CH_2)_e-$ oder eine Gruppe

$$-\overset{R_{15}}{\underset{R_{16}}{\overset{|}{\underset{|}{C}}}}-$$

ist, wobei

e 0 bis 8 ist, und

$R_{15}$ und $R_{16}$ unabhängig voneinander Wasserstoff, $C_1$-$C_4$ Alkyl, Allyl, Benzyl, Cyanomethyl, Cyanoäthyl oder eine Gruppe $-CH_2COOR_{17}$, wobei

$R_{17}$ Methyl oder Aethyl ist, bedeuten, oder

$Z_2$ eine Gruppe der Formeln IX, X oder XI ist,

r 1 oder 2 ist, wobei der Quotient q/r 1, 1.5, 2 oder 3 ist, und

L eine Gruppe der Formel II ist, worin

$R_1$ Wasserstoff oder Methyl ist, und

$Z_1$ Wasserstoff, $C_1$-$C_4$ Alkyl, Allyl, $C_2$-$C_6$ Alkoxyalkyl, Benzyl, Acetyl oder eine der Gruppen $-CH_2COOR_2$ oder $-CH_2-CH(R_3)-OR_4$ oder eine Gruppe der Formel III bedeutet, wobei

$R_2$ $C_1$-$C_4$ Alkyl ist, und

$R_3$ Wasserstoff ist, und

$R_4$ eine aliphatische Acylgruppe mit 1-8 C-Atomen ist, und

$R_6$ eine Gruppe der Formel V bedeutet, worin $R_9$ Wasserstoff ist, und

$R_7$ Wasserstoff ist, und

$R_8$ Wasserstoff oder Methyl bedeutet, und

a 0, 1 oder 2 ist, und

b 0 oder 1 ist, und

c 0 oder, falls a und/oder b von 0 verschieden sind, auch 1 ist, und

X -O- ist, und

Y -O- oder $-\dot{N}-R_{10}$ bedeutet, wobei

$R_{10}$ Wasserstoff oder Methyl, oder, falls a, b und c 1 und Y -O- sind, auch eine Gruppe $-CH_2-CH_2-OR_6$, worin $R_6$ die oben angegebene Bedeutung hat, ist, und

$Z_2$ Wasserstoff, $C_2$-$C_7$ aliphatisches Acyloxy, gegebenenfalls mit Methyl oder Methoxy monosubstituiertes Benzoyloxy, oder eine gegebenenfalls mit Methyl N-substituierte $C_2$-$C_7$ Acylamidogruppe ist oder eine der Gruppen $-(CH_2)_d-R_{12}$,

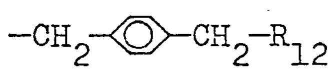 oder 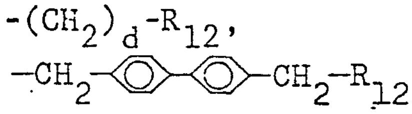 ist,

$Z_2$ und $Z_3$ zusammen eine der Formeln XV, XVI oder XVII bedeuten, worin $R_6$ die oben angegebene Bedeutung hat, und

$R_{24}$ Wasserstoff, Methyl oder Aethyl ist und

$R_{25}$ Wasserstoff, $C_1$-$C_{12}$ Alkyl, $C_3$-$C_8$ Alkenyl, eine Gruppe $-CH_2COOR_{26}$ oder $-CH_2-CH(R_8)-OR_6$ bedeutet, wobei $R_6$ und $R_8$ die oben angegebene Bedeutung haben, und

$R_{26}$ $C_1$-$C_{12}$ Alkyl ist, und

m 0, 1 oder 2 ist, und

A $H_2O$ oder ein Amin der Formel XVIII ist, worin

$R_{27}$ gegebenenfalls mit -OH substituiertes $C_1$-$C_{18}$ Alkyl, $C_5$-$C_{12}$ Cycloalkyl, gegebenenfalls mit $C_1$-$C_{12}$ Alkyl substituiertes $C_6$-$C_{10}$ Aryl, $C_7$-$C_{20}$ Aralkyl, eine gegebenenfalls substituierte Aminoalkylgruppe mit 1-8 C-Atomen im Alkylteil oder Piperidinylgruppe ist, und

$R_{28}$ Wasserstoff, oder gegebenenfalls mit -OH substituiertes $C_1$-$C_{18}$ Alkyl, $C_5$-$C_{12}$ Cycloalkyl oder eine gegebenenfalls substituierte Aminoalkylgruppe mit 1-8 C-Atomen im Alkylteil oder Piperidinylgruppe bedeutet, oder

$R_{27}$ und $R_{28}$ zusammen mit dem N-Atom einen durch Alkylgruppen substituierten oder unsubstituierten Pyrrolidin-, Piperidin- oder Morpholinring bilden, und

$R_{29}$ Wasserstoff oder gegebenenfalls mit -OH substituiertes $C_1$-$C_{18}$ Alkyl bedeutet.

4. Verbindungen gemäss Anspruch 1 der Formel I, worin

$M^{q\oplus}$ $Ca^{2+}$, $Mg^{2+}$, $Zn^{2+}$, $Co^{2+}$, $Ni^{2+}$ oder $Al^{3+}$ ist, und

q 2 oder 3 ist, und

$R_{10}^{**}$ Wasserstoff, $C_1$-$C_4$ Alkyl oder Phenyl ist, oder

$Z_2$ ferner eine der Gruppen XIII oder XIV ist, worin

$R_6$ die oben angegebene Bedeutung hat, und

$R_{22}$ und $R_{23}$ unabhängig voneinander Wasserstoff, Methyl oder Aethyl sind, und

$Z_3$ Wasserstoff ist, oder

$Z_2$ und $Z_3$ zusammen eine der Formeln XV, XVI oder XVII bedeuten, worin $R_6$ die oben angegebene Bedeutung hat, und

$R_{24}$ Wasserstoff oder Methyl ist, und

$R_{25}$ $C_1$-$C_8$ Alkyl, Allyl, oder eine der Gruppen $-CH_2-COOR_{26}$ oder $-CH_2-CH(R_8)-OR_6$ bedeutet, worin $R_6$ und $R_8$ die oben angegebene Bedeutung haben, und

$R_{26}$ $C_1$-$C_8$ Alkyl ist, und

m 0, 1 oder 2 ist, und

A $H_2O$ oder ein Amin der Formel XVIII ist, worin

$R_{27}$ und $R_{28}$ unabhängig voneinander $C_1$-$C_8$ Alkyl, $C_1$-$C_6$ Hydroxyalkyl oder eine substituierte Aminoalkylgruppe der Formel VIIb

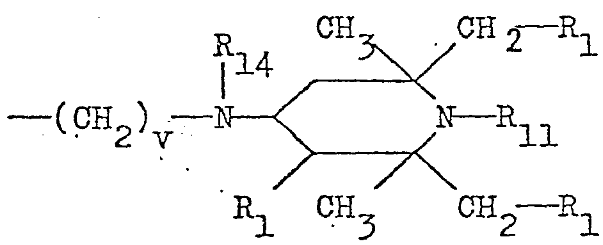

(VIIb)

ist, worin $R_1$, $R_{11}$ und $R_{14}$ die angegebene Bedeutung haben und v 1 bis 8 ist, oder

$R_{27}$ und $R_{28}$ zusammen mit dem N-Atom eine Piperidingruppe bilden, und

$R_{29}$ Wasserstoff, $C_1$-$C_8$ Alkyl oder $C_1$-$C_6$ Hydroxyalkyl ist.

worin d 2 bis 6 ist, und

$R_{12}$ eine Gruppe der Formel VII ist, worin $R_1$ und $Z_1$ die oben angegebene Bedeutung haben, oder

$Z_2$ ferner eine Gruppe der Formel VIII bedeutet, worin

X' -O- oder $-\overset{|}{N}-R_{14}$ ist, wobei

$R_{14}$ Wasserstoff oder Methyl ist, und

$R_{13}$ $-(CH_2)_e-$ oder eine Gruppe $-\underset{R_{16}}{\overset{R_{15}}{\overset{|}{\underset{|}{C}}}}-$ ist, wobei

e 0 bis 8 ist, und

$R_{15}$ und $R_{16}$ unabhängig voneinander Wasserstoff, $C_1$-$C_4$ Alkyl, Allyl, Benzyl oder Cyanomethyl sind, oder

$Z_2$ ferner eine Gruppe der Formel XI ist, worin

$R_{20}$ eine Gruppe derFormel V mit oben angegebener Bedeutung ist, und

$R_{21}$ $C_2$-$C_6$ Alkylen oder Phenylen bedeutet, oder

$Z_2$ ferner eine Gruppe der Formel XIIa ist, worin a, b, c, $R_6$ und $R_8$ oben angegebene Bedeutung haben, und

f 0 oder 1 ist, und

$X_1$ -O- oder $-\overset{|}{N}-R^*_{10}$ bedeutet, worin

$R^*_{10}$ Wasserstoff, $C_1$-$C_8$ Alkyl, Cyclohexyl, $C_3$-$C_8$ Alkoxyalkyl, $C_7$-$C_8$ Aralkyl, gegebenenfalls mit $C_1$-$C_3$ Alkyl und/oder $C_1$-$C_2$ Alkoxy monosubstituiertes Phenyl oder falls a, b und c 1 und $Y_1$ -O- sind, auch eine Gruppe $-CH_2-CH_2-OR_6$, worin $R_6$ die oben angegebene Bedeutung hat, ist, und $R^*_{10}$ ferner eine Gruppe VI bedeutet, worin

$R_1$ die oben angegebene Bedeutung hat, und

$R_{11}$ Wasserstoff oder Methyl ist, und

$Y_1$ -O- oder $-\overset{|}{N}-R^{**}_{10}$ bedeutet, worin

$R_{13}$ $-(CH_2)_e-$ oder eine Gruppe $-\overset{R_{15}}{\underset{R_{16}}{C}}-$ ist, wobei

e 0 bis 8 ist, und

$R_{15}$ und $R_{16}$ unabhängig voneinander Wasserstoff, $C_1$-$C_4$ Alkyl, Allyl oder Benzyl sind, oder

$Z_2$ ferner eine Gruppe der Formel XI ist, worin

$R_{20}$ eine Gruppe der Formel V mit oben angegebener Bedeutung ist, und

$R_{21}$ $C_2$-$C_6$ Alkylen ist, oder

$Z_2$ ferner eine Gruppe der Formel XIIa ist, worin a, b, c, $R_6$ und $R_8$ oben angegebene Bedeutung haben, und

f 0 oder 1 ist, und

$X_1$ -O- oder $-\overset{|}{N}-R^*_{10}$ bedeutet, worin

$R^*_{10}$ Wasserstoff, $C_1$-$C_8$ Alkyl, $C_3$-$C_6$ Alkoxyalkyl, $C_7$-$C_8$ Aralkyl, gegebenenfalls mit Methyl, Methoxy oder Aethoxy monosubstituiertes Phenyl oder eine Gruppe VI bedeutet, worin

$R_1$ die oben angegebene Bedeutung hat, und

$R_{11}$ Wasserstoff oder Methyl ist, und

$Y_1$ -O- ist, oder

$Z_2$ ferner eine der Gruppen XIII oder XIV ist, worin $R_6$ die oben angegebene Bedeutung hat, und

$R_{22}$ und $R_{23}$ unabhängig voneinander Wasserstoff oder Methyl sind, und

$Z_3$ Wasserstoff ist, oder

$Z_2$ und $Z_3$ zusammen eine der Formeln XV, XVI oder XVII bedeuten, worin $R_6$ die oben angegebene Bedeutung hat, und

$R_{24}$ Wasserstoff ist, und

$R_{25}$ $C_1$-$C_8$ Alkyl, oder eine der Gruppen $-CH_2COOR_{26}$

5. Verbindungen gemäss Anspruch 1 der Formel I,

worin

$M^{q+}$ $Ca^{2+}$, $Mg^{2+}$, $Zn^{2+}$, $Co^{2+}$, $Ni^{2+}$ oder $Al^{3+}$ ist, und

q 2 oder 3 ist, und

r 1 oder 2 ist, wobei der Quotient q/r 1, 1.5, 2 oder 3 ist, und

L eine Gruppe der Formel II ist, worin

$R_1$ Wasserstoff ist, und

$Z_1$ Wasserstoff, Methyl, Acetyl oder eine Gruppe der Formel III bedeutet, wobei

$R_6$ eine Gruppe der Formel V bedeutet, worin $R_9$ Wasserstoff ist, und

$R_7$ Wasserstoff und

$R_8$ Wasserstoff oder Methyl ist, und

a 0, 1 oder 2 ist, und

b 0 oder 1 und

c 0 oder, falls a und/oder b von 0 verschieden sind, auch 1 ist, und

X -O- ist, und

Y $-\overset{|}{N}-R_{10}$ bedeutet, wobei

$R_{10}$ Wasserstoff ist, und

$Z_2$ Wasserstoff, $C_2$-$C_7$ aliphatisches Acyloxy, Benzoyloxy, eine gegebenenfalls mit Methyl N-substituierte Acylamidogruppe ist, oder eine der Gruppen $-(CH_2)_d-R_{12}$, $-CH_2-$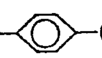$-CH_2-R_{12}$ oder $-CH_2-$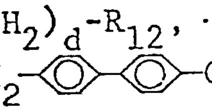$-CH_2-R_{12}$ ist, worin d 2 bis 6 ist, und

$R_{12}$ eine Gruppe der Formel VII ist, worin $R_1$ und $Z_1$ die oben angegebene Bedeutung haben, oder

$Z_2$ ferner eine Gruppe der Formel VIII ist, worin

X' -O- oder $-\overset{|}{N}-R_{14}$ bedeutet, wobei

$R_{14}$ Wasserstoff oder Methyl ist, und

0000487

Europäisches Patentamt

# EUROPÄISCHER RECHERCHENBERICHT

Nummer der Anmeldung

EP 78 10 0326

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| D | DE - A - 2 625 967 (CIBA GEIGY)<br>* Anspruch 1 *<br>--<br>NL - A - 76 14548 (CIBA GEIGY)<br>* Seite 23; Beispiel 1 *<br>---- | 1-10<br><br><br>1-10 | C 07 D 211/00<br>C 07 D 491/00<br>C 08 K 5/00<br>C 08 L 23/06<br>C 07 D 401/00 |
| | | | RECHERCHIERTE SACHGEBIETE (Int. Cl.²) |
| | | | C 08 K 5/00<br>C 08 K 5/34 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsatze<br>E: kollidierende Anmeldung<br>D: in der Anmeldung angeführtes Dokument<br>L: aus andern Gründen angeführtes Dokument |
| ☒ | Der vorliegende Recherchenbericht wurde für alle Patentansprüche erstellt | | &: Mitglied der gleichen Patentfamilie, übereinstimmendes Dokument |

| Recherchenort | Abschlußdatum der Recherche | Prüfer |
|---|---|---|
| Den Haag | 20-10-1978 | [illegible] |

EPA form 1503.1 06.78